(19) Europäisches Patentamt
European Patent Office
Office européen des brevets

(11) Veröffentlichungsnummer: **0 268 554 B1**

(12) # EUROPÄISCHE PATENTSCHRIFT

(45) Veröffentlichungstag der Patentschrift: **27.12.91**

(21) Anmeldenummer: **87810598.0**

(22) Anmeldetag: **16.10.87**

(51) Int. Cl.5: **C07D 231/14**, C07D 405/12, C07D 409/12, C07D 401/12, C07D 417/12, C07D 487/04, A01N 43/56, A01N 43/54, A01N 43/78

(54) **1,5-Diphenylpyrazol-3-carbonsäurederivate zum Schützen von Kulturpflanzen.**

(30) Priorität: **22.10.86 CH 4215/86**
**22.10.86 CH 4217/86**

(43) Veröffentlichungstag der Anmeldung:
**25.05.88 Patentblatt 88/21**

(45) Bekanntmachung des Hinweises auf die Patenterteilung:
**27.12.91 Patentblatt 91/52**

(84) Benannte Vertragsstaaten:
**AT BE CH DE ES FR GB GR IT LI LU NL SE**

(56) Entgegenhaltungen:
**EP-A- 0 189 303**
**EP-A- 0 202 169**
**DE-A- 2 144 568**
**DE-A- 2 144 568**
**DE-A- 2 701 097**
**DE-A- 3 525 568**
**US-A- 33 182**
**US-A- 3 899 508**
**US-A- 4 116 673**
**US-A- 4 245 106**
**US-A- 4 639 266**

**Chem. Ber. 112 (1979) 1197**

**Monatshefte der Chemie 90 (1967) 1618-1650**

**Il Formoco Ed Sc. 40 (4) (1984) 272-284**

**J. Chem. and Eng. Data 22 (1977) 104-110**

**Chem. Abs. 64 (1966) 17572f**

(73) Patentinhaber: **CIBA-GEIGY AG**
**Klybeckstrasse 141**
**CH-4002 Basel(CH)**

(72) Erfinder: **Moser, Hans, Dr.**
**Maispracherstrasse 12**
**CH-4312 Magden(CH)**
Erfinder: **Böhner, Beat, Dr.**
**Hügelweg 3**
**CH-4102 Binningen(CH)**
Erfinder: **Föry, Werner, Dr.**
**Inzlingerstrasse 11**
**CH-4125 Riehen(CH)**

**EP 0 268 554 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue 1,5-Diphenylpyrazol-3-carbonsäurederivate und deren Verwendung zum Schützen von Kulturpflanzen gegen die schädigende Wirkung von herbizid wirksamen Phenoxyalkancarbonsäureestern sowie herbizide Mittel, welche diese 1,5-Diphenylpyrazol-3-carbonsäurederivate oder eine Kombination von Herbizid und 1,5-Diphenylpyrazolcarbonsäurederivat als Antagonist enthält. Ferner betrifft die Erfindung die neuen 1,5-Diphenylpyrazolcarbonsäurederivate und deren Herstellung.

Beim Einsatz von Phenoxyalkancarbonsäure-Herbiziden wie beispielsweise Phenoxyphenoxy- und Pyridyloxyphenoxy-propionsäure-Derivaten können in Abhängigkeit von Faktoren wie beispielsweise Dosis des Herbizids und Applikationsart, Art der Kulturpflanze, Bodenbeschaffenheit und klimatischen Bedingungen, wie beispielsweise Belichtungsdauer, Temperatur und Niederschlagsmengen, die Kulturpflanzen in erheblichem Masse geschädigt werden. Insbesondere kann es zu starken Schädigungen kommen, wenn im Rahmen der Fruchtfolge nach Kulturpflanzen, die gegen die Herbizide resistent sind, andere Kulturpflanzen angebaut werden, welche keine oder nur unzureichende Resistenz gegenüber den Herbiziden aufweisen.

Es wurde nun gefunden dass überraschenderweise ein Schutz von Kulturpflanzen gegen Schäden, welche durch herbizid wirksame Phenoxy-propionsäure-Derivate verursacht werden, durch Behandlung der Kulturpflanzen, von Teilen dieser Pflanzen oder von für den Anbau der Kulturpflanzen bestimmten Böden mit einem Safener aus einer Gruppe von 1,5-Diphenylpyrazol-3-carbonsäure-derivaten erzielt werden kann. Die herbizide Wirkung gegenüber Unkräutern und Ungräsern wird durch diese Derivate nicht aufgehoben.

1,5-Diphenylpyrazol-3-carbonsäurederivate, welche zum Schützen von Kulturpflanzen vor schädigenden Wirkungen herbizid wirksamer Phenoxyphenoxy-, Pyridin-2-yloxyphenoxy-, Benzoxazyloxyphenoxy-, Benzthiazolyloxyphenoxy- oder Chinoxalinyloxyphenoxypropionsäure Derivate geeignet sind, entsprechen der Formel I

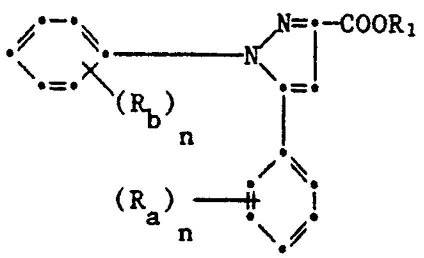

I

worin $R_a$ und $R_b$ unabhängig voneinander je Halogen, $C_1$-$C_5$-Alkyl, $C_1$-$C_5$-Halogenalkyl, $C_2$-$C_5$-Alkenyl, $C_2$-$C_5$-Alkinyl oder Cyano, n Null oder eine Zahl von 1 bis 3 und die Gruppe -$OR_1$ Hydroxy, einen Salz-oder einen beliebigen Esterrest bedeutet.

Insbesondere bedeuten im Rest -$OR_1$

$R_1$ Wasserstoff, ein pflanzenphysiologisch verträgliches Metall- oder Ammoniumkation, einen Rest $C_1$-$C_{18}$-Alkyl oder $C_3$-$C_{18}$-Cycloalkyl, der unsubstituiert oder ein- oder mehrfach durch $C_2$-$C_8$-Alkenyl, $C_2$-$C_8$-Alkinyl, $C_3$-$C_8$-Cycloalkenyl, Halogen, Nitro, Cyan, einen Rest -$XR_{10}$, -$CXR_{10}$, -CX-$XR_{10}$, -$CXNR_3R_4$, -$XCXR_{10}$, -$XCXNR_3R_4$, -$NR_3R_4$, -$NR_3CONR_3R_4$, -$CONR_3$-$NR_3R_4$, -$CONR_3$-$NR_3COR_4$, Si($C_1$-$C_4$Alkyl)$_3$, -C-($OR_7$)($OR_8$)$OR_9$, PO($R_5$)$R_6$ substituiert ist; einen Heterocyclus, Q der über C oder N gebunden ist; einen unsubstituierten oder substituierten Phenyl-oder Naphthylrest U, einen Rest-E-U oder einen Imidorest -N = C($R_2$)$R_2$,

$R_2$ $C_1$-$C_4$-Alkyl, $C_3$-$C_7$-Cycloalkyl oder die beiden $R_2$ zusammen einen 4-6 gliedrigen $C_4$-$C_{12}$-Alkylenrest der verzweigt und/oder durch Sauerstoff oder Schwefel unterbrochen sein kann

$R_3$ und $R_4$ unabhängig voneinander je Wasserstoff, $C_1$-$C_8$-Alkyl oder $C_3$-$C_8$-Cycloalkyl, unsubstituiert oder substituiert durch $C_2$-$C_6$-Alkenyl, $C_2$-$C_6$-Alkinyl, $C_3$-$C_7$-Cycloalkyl, Halogen, $C_1$-$C_8$-Alkoxy, $C_2$-$C_8$-Alkoxyalkoxy, $C_1$-$C_8$-Alkylthio, Cyan, eines von $C_3$ und $C_4$ auch $C_1$-$C_8$-Alkoxy, -$COOR_{10}$, -$CONH_2$ -CONH($C_1$-$C_4$-Alkyl), CON($R_2$)$R_2$, -$NH_2$, -NH($C_1$-$C_2$-alkyl)-N($R_2$)$R_2$ einen Heterocyclus Q einen Phenyl- oder Napthylrest U, der direkt oder über eine $C_1$-$C_{14}$-Alkylenbrücke an das Stickstoffatom gebunden ist,

$R_3$ und $R_4$ zusammen eine 4-6-gliedrige $C_4$-$C_{12}$-Alkylen- oder Alkenylenkette, die verzweigt und/oder durch Sauerstoff oder Schwefel N($C_1$-$C_4$-Alkyl), N(Benzyl), -$SO_2$- oder -CO- oder -C($OR_7$)$OR_8$ unterbrochen sein kann und die durch Halogen, $C_1$-$C_4$-Alkyl, $C_1$-$C_4$-Alkoxy, -$NH_2$, -NH($C_1$-$C_4$-Alkyl), -NH($C_1$-$C_4$-Alkyl)$_2$ substituiert sein kann,

$R_5$ und $R_6$ unabhängig voneinander je $C_1$-$C_4$-Alkyl, Hydroxy oder $C_1$-$C_4$-Alkoxy

$R_7$ und $R_8$ unabhängig voneinander je $C_1$-$C_4$-Alkyl,

$R_7$ und $R_8$ zusammen $C_2$-$C_4$-Alkylen
$R_9$ und $R_{10}$ je Wasserstoff, $C_1$-$C_8$-Alkyl oder $C_3$-$C_8$-Cycloalkyl unsubstituiert oder substituiert durch $C_2$-$C_6$-Alkenyl, $C_2$-$C_6$-Alkinyl oder $C_3$-$C_6$-Cycloalkenyl, Halogen, $C_1$-$C_4$-Alkoxy, $C_2$-$C_8$-Alkoxyalkoxy, $C_1$-$C_6$-Alkoxycarbonyl, $C_1$-$C_6$-Haloalkoxycarbonyl, einen Heterocyclus Q oder einen Phenyl- oder Naphthylrest U.
$R_9$ und $R_{10}$ bedeuten ferner den Phenyl- oder Naphthylrest U oder einen über C-gebundenen Heterocyclus Q,
Q ein gesättigten oder ungesättigten 5-12-gliedrigen Heterocyclus, der 1-4 Heteroatome N, O, S resp. eine -SO-, -$SO_2$-, N($C_1$-$C_4$-Allyl)-oder -N(Benzyl)-Gruppe enthält und der durch Halogen, $C_1$-$C_4$-Alkyl, $C_1$-$C_4$-Alkoxy, $C_1$-$C_4$-Haloalkyl, $C_1$-$C_4$-Haloalkoxy, Cyan, Nitro substituiert sein kann,
E eine $C_1$-$C_4$-Alkylen-, $C_3$-$C_4$-Alkenylen- oder $C_3$-$C_4$-Alkinylenbrücke,
U ein Phenyl- oder Naphthylrest, der unsubstituiert oder ein oder mehrfach substituiert ist durch Halogen, $C_1$-$C_4$-Alkyl, X $C_1$-$C_4$-Alkyl, $C_1$-$C_4$-Alkylsulfinyl, $C_1$-$C_4$-Alkylsulfonyl, $C_1$-$C_4$-Haloalkyl, $C_1$-$C_4$-Haloalkoxy, Cyano, Nitro, -COOH, -COO$C_1$-$C_4$-Alkyl, -CO$C_1$-$C_4$-Alkyl, $CONH_2$, CONH($C_1$-$C_4$-Alkyl), CON($C_1$-$C_4$-Alkyl)$_2$, -$SO_2NH_2$, $SO_2$NH($C_1$-$C_4$-Alkyl), $SO_2$N($C_1$-$C_4$-Alkyl), Pyrrolidino, Piperidino oder durch den Pyrrolidinocarbonyl, Piperidinocarbonyl, Morpholinocarbonylrest,
X Sauerstoff oder Schwefel
bedeuten.

Unter pflanzenphysiologisch verträglichen Metall- und Ammoniumkationen werden die Kationen von in Herbiziden üblichen Salzen verstanden, wie Alkalimetall-, Erdalkalimetall-, Eisen-, Kupfer-, Mangan- oder Ammonium-Alkylammonium-, Hydroxylalkyl- oder Alkoxyalkylammonium-Kationen.

Unter Alkylresten werden Reste mit der angegebenen Anzahl Kohlenstoffatomen verstanden. Diese Reste können geradkettig oder verzweigt sein. Die gebräuchlichsten Reste sind beispielsweise Methyl, Aethyl, n-Propyl, Isopropyl, n-Butyl, Isobutyl, sek.Butyl, tert.Butyl, n-Pentyl, Isopentyl, n-Hexyl und n-Octyl. Die Alkenyl-und Alkinylreste können ebenfalls geradkettig oder verzweigt sein und umfassen 3 bis 6 Kohlenstoffatome. Die am verbreitesten Reste sind beispielweise Allyl, Methallyl, Buten, Butadien, Propinyl, Methylpropinyl, 1-Butinyl, 2-Butinyl. Cycloalkyl- oder Cycloalkenylreste haben vorzugsweise 3 - 12 Kohlenstoffatome, sie können auch benzanneliert sein. Typische Vertreter sind beispielsweise Cyclopropyl, Cyclopentyl, Cyclohexyl, Cyclohexenyl, Indan, Tetrahydronaphthalin, Decalin. Unter Halogen wird Fluor, Chlor, Brom, Jod, insbesondere Fluor und Chlor verstanden. Halogenalkyl- und Halogenalkenyl-Reste können ein- oder mehrfach mit Halogen substituiert sein.

Der Rest Q bedeutet einen gesättigten oder ungesättigten 5 bis 12-gliedrigen Heterocyclus der noch ein zwei oder drei weitere Heteroatome oder eine Sulfinyl- resp. Sulfonylgruppe enthält, durch ein oder zwei Carbonylgruppen unterbrochen sein kann, und welcher benzannelliert, unsubstituiert oder substituiert sein kann.

Als Heteroatome kommen dabei ein zwei oder drei weitere Stickstoffatome, bis zwei Schwefel- oder Sauerstoffatome in Frage, wobei 2 Sauerstoffatome nicht direkt benachbart sein können.

Beispiele für solche Heterocyclen, sind untenstehend aufgeführt: Pyrrolin, Pyrrolidin, Imidazolin, Imidazolidin, Pyrazolin, Pyrazolidin, Isoxazolin, Isoxazolidin, Oxazolin, Oxazolidin, Isothiazolidin, Thiazoline, Thiazolidine, Dithiazolidine, Oxadiazolidine, Pyridin, Piperidin, Piperazin, Tetrahydropyrimidin und -pyrazin, Pyrimidin, Pyridazin, Morpholin, Thiomorpholin, Thiazine, Hexahydrotriazine, Tetrahydrotriazine, Oxadiazine, Oxatriazine, Hexahydroazepin, Hexahydrodiazepine, Diazepine, Hexahydrooxazepine, Azacyclooctan, Benzofuran, Benzthiophen, Indol, Indolin, Isoindolin, Benzimidazolin, Benzindazolin, Benzoxazolin, Benzthiazoline, Benzisoxazolin, Benztriazol, Chinolin, Tetrahydrochinolin, Tetrahydroisochinolin, Chinazolin, Chinoxalin, Phtalazin, Benzmorpholin, Benzothiomorpholin, Tetrahydrobenzazepine und -diazepine, Tetrahydrobenzoxazepine, 1,5-diazabicyclo[4,3,0]-nonane, Dihydrobenzoxazine, 1,6-diazabicyclo[5,3,0]decane, 1,4-diazabicyclo[3,3,0]octane, 1,5-diazabicyclo[4,4,0]decane.

Oben erwähnte Heterocyclen können auch die Bedeutung von Substituenten haben. Weitere Beispiele von heterocyclischen Systemen mit Substituentenfunktion sind beispielsweise Pyrrol, Imidazol, Pyrazol, Isoxazol, Oxazol, Isothiazol, Thiazol, Triazole, Oxadiazole, Thiadiazole, Tetrazole, Oxatriazole, Thiatriazole, Furan, Tetrahydrofuran, Dioxole, Dioxolane, Oxathiole, Oxathiolane, Thiophen, Tetrahydrothiophen, Dithiolane, Dithiazole, Pyridin, Pyrane, Thiopyrane, Pyridazin, Pyrimidin, Pyrazin, Tetrahydropyran, Tetrahydrothiopyran, Dioxine, Dioxane, Dithiine, Dithiane, Oxazine, Thiazine, Oxathiine, Oxathiane, Triazine, Oxadiazine, Thiadiazine, Oxathiazine, Dioxazine, Azepine, Oxepine, Thiepine, Diazepine, Oxazepine, Indole, Benzofurane, Benzothiophene, Indazole, Benzimidazole, Benzdioxole, Benzdithiole, Benzisoxazole, Benzthiazole, Benzoxazole, Benzoxathiole, Benztriazole, Benzoxadiazole, Benzofurazan, Benzothiadiazole, Chinolin, Isochinolin, Chromene, Chroman, Isochromen, Isochroman, Thiochromene, Isothiochromene, Thiochroman, Isothiochroman, Cinnolin, Chinazolin, Chinoxalin, Phtalazin, Benzdioxine, Benzdithiine, Benzoxazine, Benzdioxane, Benzoxathiane, Benzotriazine, Benzazepine, Benzdilazepine, Benzoxazepine, Purine, Pteridine,

Phenoxazine, Phenothiazine.

Die heterocylcischen Reste können substituiert sein durch Halogen, $C_1$-$C_4$-Alkyl, $C_1$-$C_4$-Alkoxy, $C_1$-$C_4$-Haloalkyl, $C_1$-$C_4$-Haloalkoxy, Cyan oder Nitro.

Die Pyrazol-Derivate der Formel I besitzen in hervorragendem Masse die Fähigkeit, Kulturpflanzen gegen die schädigende Wirkung von herbizid wirksamen 2-[4-(Phenoxy-, Pyridin-2-yl-, Benzoxazol-, Benzthiazol- und Chinoxalin-2-yl-oxy)-phenoxy]-propionsäure-estern der Formel II

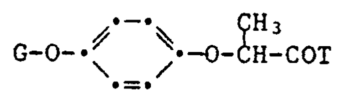

(II),

worin G

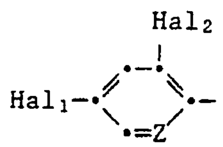

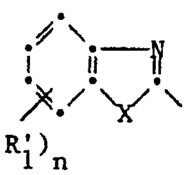

oder

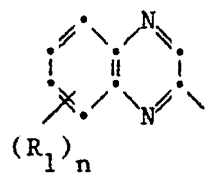

,

$Hal_1$ Fluor, Chlor, Brom, Jod oder Trifluoromethyl, $Hal_2$ Wasserstoff, Fluor, Chlor, Brom oder Trifluoromethyl, Z für Stickstoff oder Methin -CH=, X ein Sauerstoff oder Schwefelatom, $R_1'$ Halogen, Trifluormethyl, Nitro, Cyan, $C_1$-$C_4$-Alkyl oder $C_1$-$C_4$-Alkoxy und n 0, 1, 2 oder 3 bedeuten, zu schützen.

In den Verbindungen der Formel II bedeutet Halogen, Fluor, Chlor, Brom oder Jod, vorzugsweise Fluor, Chlor, und Brom.

T steht für

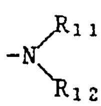

,

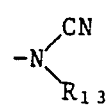

,

$-OR_{14}$ oder $-O-N=CR_{15}R_{16}$

$R_{11}$, $R_{12}$: unabhängig voneinander Wasserstoff, $C_1$-$C_8$-Alkoxy, $C_1$-$C_8$-Alkyl, Phenyl oder Benzyl, $R_{11}$ und $R_{12}$ zusammen mit dem sie tragenden Stickstoffatom einen 5-bis 6-gliedrigen gesättigten Stickstoffheterocyclus, der durch ein Sauerstoff- oder Schwefelatom unterbrochen sein kann,

$R_{13}$: $C_1$-$C_4$-Alkyl, $C_3$-$C_4$-Alkenyl, $C_3$-$C_4$-Alkinyl oder $C_2$-$C_4$-Alkoxyalkyl

$R_{14}$: Wasserstoff oder das Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, -COOR, -COSR, $-CONH_2$-, -CON($C_1$-$C_4$-alkoxy)-$C_1$-$C_4$-alkyl, -CO-N-di-$C_1$-$C_4$-alkyl, -CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest;

$C_3$-$C_9$-Cycloalkyl; oder gegebenfalls durch Cyan, $C_1$-$C_4$-Alkyl, $C_1$-$C_4$-Alkoxy, Acetyl, $-COOR_{17}$, $-COSR_{17}$, $-CONH_2$, -CON($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,

und $R_{16}$ unabhängig voneinander $C_1$-$C_4$-Alkyl oder zusammen eine 3-bis 6-gliedrige Alkylenkette und

$R_{17}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkenyl, $C_3$-$C_6$-Alkinyl oder $C_3$-$C_6$-Halogenalkinyl bedeuten.

In den Verbindungen der Formel II bedeutet Halogen als selbstständiger Substituent oder Teil eines anderen Substituenten, wie Halogenalkyl, Halogenalkoxy, Halogenalkenyl oder Halogenalkinyl, Fluor, Chlor, Brom oder Jod, worunter Fluor oder Chlor bevorzugt sind.

Alkyl steht je nach der Anzahl der vorhandenen Kohlenstoffatome für Methyl, Aethyl, n-Propyl, i-Propyl sowie die isomere Butyl, Pentyl, Hexyl, Heptyl oder Oktyl. Die in den Resten Alkoxy, Alkoxyalkyl, Halogenalkyl oder Halogenalkoxy enthaltenen Alkylgruppen haben die gleiche Bedeutung. Bevorzugt sind

jeweils Alkylgruppe mit niedriger Anzahl von Kohlenstoffatomen.

Bevorzugte Halogenalkylreste, bzw. Halogenalkylteile in Halogenalkoxyresten sind: Fluormethyl, Difluormethyl, Trifluormethyl, Chlormethyl, Trichlormethyl, 2-Fluoräthyl, 2,2,2-Trifluoräthyl, 1,1,2,2-Tetrafluoräthyl, Perfluoräthyl, 2-Chloräthyl, 2,2,2-Trichloräthyl, 2-Bromäthyl und 1,1,2,3,3,3-Hexafluorpropyl.

Cycloalkyl steht für mono-, und bi-cyclische gesättigte Kohlenwasserstoffringsysteme wie Cyclopropyl, Cyclobutyl, Cyclopentyl, Cyclohexyl, Cycloheptyl, Cyclooktyl, Cyclononyl, Bicyclo[4.3.0]nonyl, Bicyclo-[5.2.0]nonyl oder Bicyclo[2.2.2.]oktyl.

Besonders bemerkenswert ist die Schutzwirkung von Pyrazol-Derivaten der Formel I gegenüber solchen Herbiziden der Formel II, in denen T für die Gruppen -O-$R_{14}$, oder -O-N=$CR_{15}R_{16}$ steht, wobei $R_{14}$ Wasserstoff, $C_1$-$C_4$-Alkyl, $C_3$-$C_4$-Alkinyl oder durch $C_1$-$C_4$-Alkoxycarbonyl oder Di-$C_1$-$C_4$-alkylamino substituiertes $C_1$-$C_4$-Alkyl und
$R_{15}$ und $R_{16}$ unabhängig voneinander $C_1$-$C_4$-Alkyl oder
$R_{15}$ und $R_{16}$ zusammen eine $C_4$-$C_7$-Alkylenkette bedeuten.

Speziell hervorzuhebende Einzelbedeutungen für T sind dabei Methoxy, Aethoxy, Propyloxy, Isopropyloxy, Butyloxy, Dimethylaminoäthoxy, Propargyloxy, 1-Cyano-1-methyläthoxy, Methoxycarbonylmethylthio, 1-Aethoxycarbonyläthoxy, Butyloxycarbonyl, -O-N=$C(CH_3)_2$,-O-N=$C(CH_3)C_2H_5$ oder -O-N=$C(CH_2)_5$, und für G

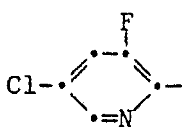 , 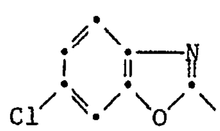 , 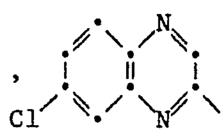 ,

besonders der
2-[4-(6-chlorbenzoxazol-2-yloxy)-phenoxy]-propionsäure-äthylester, der
2-[4-(6-chlorchinoxalin-3-yloxy)-phenoxy]-propionsäure-äthylester, sowie die
2-[4-(5-Chlor-3-fluorpyridin-2-yloxy)-phenoxy]-propionsäurederivate der Tabelle 1.

Tabelle 1

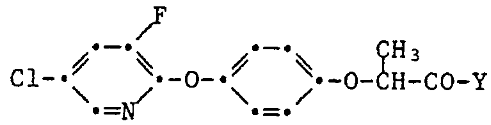

(II)

| Nr. | Y | physikalische Konstante |
|---|---|---|
| 2.1 | $-OCH_3$ | Smp. 63-64°C |
| 2.2 | $-OC_4H_9-n$ | $n_D^{35} = 1.5275$ |
| 2.3 | $-O-N=C(CH_3)_2$ | $n_D^{35} = 1.5488$ |
| 2.4 | $-OC_2H_5$ | $n_D^{35} = 1.5358$ |
| 2.5 | $-O-CH_2-CH_2-N(CH_3)_2$ | $n_D^{35} = 1.5334$ |
| 2.6 | $-O-CH_2-C\equiv CH$ | $n_D^{35} = 1.5492$ |
| 2.7 | 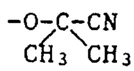 | $n_D^{35} = 1.5330$ |
| 2.8 | $-S-CH_2-COOCH_3$ | $n_D^{35} = 1.5607$ |
| 2.9 | 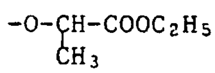 | $n_D^{35} = 1.5227$ |
| 2.10 | $-O-CH_2-COOC_4H_9-n$ | $n_D^{35} = 1.5223$ |
| 2.11 | $-OC_3H_7-n$ | $n_D^{35} = 1.5319$ |
| 2.12 | $-OC_3H_7-i$ | $n_D^{35} = 1.5284$ |
| 2.13 | 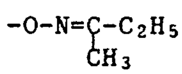 | $n_D^{35} = 1.5340$ |
| 2.14 | 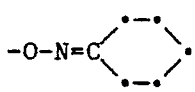 | $n_D^{35} = 1.5360$ |
| 2.15 | $-OCH_3$ (2R) | $n_D^{35} = 1.5359$ |
| 2.16 | -OH | Smp. 95-97°C |
| 2.17 | $-S-CH_2-COOCH_3$ (2R) | $n_D^{35} = 1.5623$ |

Tabelle 1 (Fortsetzung)

| Nr. | Y | physikalische Konstante |
|---|---|---|
| 2.18 | $-O-CH-COOC_2H_5$ (2R,S)<br>$CH_3$ | $n_D^{35} = 1,5223$ |
| 2.19 | $-O-CH_2-C{\equiv}CH$ (2R) | Smp. 55-56°C |
| 2.20 | $-NH-OCH_3$ | Smp. 103-105°C |

ganz besonders ist dabei die Verbindung 2.19

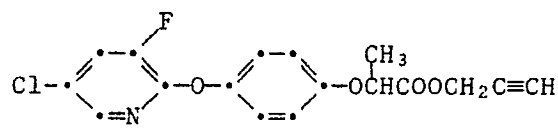

oder sein 2R Enantiomer hervorzuheben.

Das optisch aktive Kohlenstoffatom der Propionsäuregruppe kann sowohl in der R- also auch S-Konfiguration vorliegen. Ohne besondere Angabe sind hierin die racemischen Gemische gemeint. Bevorzugte Herbizide der Formel II sind 2R-konfiguriert.

Als Kulturpflanzen, welche durch 1,5-Diphenylpyrazol-3-carbonsäurederivate der Formel I gegen schädigende Wirkungen von Herbiziden der Formel II geschützt werden können, kommen insbesondere diejenigen in Betracht, die auf dem Nahrungs- oder Textilsektor von Bedeutung sind, beispielsweise Zuckerrohr und insbesondere Kulturhirse, Mais, Reis und andere Getreidearten (Weizen, Roggen, Gerste, Hafer).

Gegenstand der Erfindung sind auch die neuen 1,5-Diphenylpyrazol-3-carbonsäurederivate der Formel I

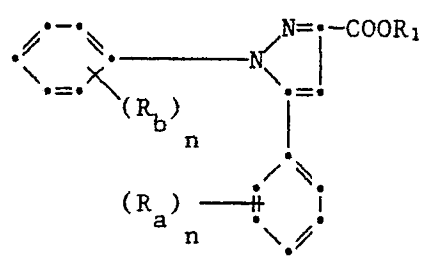

I

worin $R_a$ und $R_b$ unabhängig voneinander je Halogen, $C_1$-$C_5$-Alkyl, $C_1$-$C_5$-Halogenalkyl, $C_2$-$C_5$-Alkenyl, $C_2$-$C_5$-Alkinyl oder Cyano, n Null oder eine Zahl von 1 bis 3 und $R_1$ die oben gegebene Bedeutung hat, mit der Massgabe, dass wenn ($R_a$) Wasserstoff, n = 1 und $R_1$ Wasserstoff oder Aethyl bedeuten, $R_b$, falls es, Methyl oder Halogen bedeutet, in der ortho-Stellung sein muss.

Darunter sind diejenigen von Bedeutung, in denen $R_a$, $R_b$ und n die unter der Formel I gegebene Bedeutung haben, während $R_1$ $C_1$-$C_{18}$-Alkyl oder $C_3$-$C_{18}$-Cycloalkyl unsubstituiert oder ein- oder mehrfach substituiert durch $C_2$-$C_8$-Alkenyl, $C_2$-$C_8$-Alkinyl, $C_3$-$C_8$-Cycloalkenyl, Halogen, Nitro, Cyan, $C_1$-$C_4$-Alkoxy, $C_1$-$C_4$-Alkoxycarbonyl, $C_1$-$C_4$-Alkylcarbonyl, Carbamoyl, Di($C_1$-$C_4$-Alkoxycarbamoyl), Amino, $C_1$-$C_4$-Alkylamino, Di($C_1$-$C_4$-Alkylamino) einen 5-6-gliedrigen Heterocylus Q der über N oder C gebunden ist oder einen Phenyl- oder Napthylrest U oder einen Imidorest $-N=C(R_2)R_2$ wobei $R_2$ Q und U die unter der Formel I gegebene Bedeutung haben mit der Massgabe, dass wenn $(R_a)_n$ Wasserstoff, n = 1 und $R_1$ Wasserstoff sind, $(R_b)_n$ falls es Methyl oder Halogen bedeutet, in der ortho-Stellung sein muss.

Sehr gute Wirkung zeigen die 1,5-Diphenylpyrazol-3-carbonsäureester der Formel I worin $R_a$, $R_b$ und n die unter der Formel I gegebene Bedeutung haben während $R_1$ $C_1$-$C_{18}$-Alkyl unsubstituiert oder ein-oder mehrfach substituiert durch $C_2$-$C_8$-Alkyl, $C_2$-$C_8$-Alkinyl, Halogen, Nitro, Cyan, $C_1$-$C_4$-Alkoxy oder Phenyl

bedeutet, darunter besonders die 1,5-Diphenylpyrazol-3-carbonsäuresalze der Formel I worin $R_a$, $R_b$ und n die unter der Formel I gegebene Bedeutung haben und $R_1$ ein pflanzenphysiologisch verträgliches Metall- oder Ammoniumkation bedeutet, insbesondere 1-(2-Chlorphenyl)-3-methoxycarbonyl-5-phenylpyrazol, 1-(2,4-Dichlorphenyl)-3-methoxycarbonyl-5-phenylpyrazol und 1-(2-Chlorphenyl-3-methoxycarbonyl-5-(2-fluorphenyl)pyrazol.

Die Säurehalogenide der 1,5-Diphenylpyrazol-3-carbonsäuren der Formel I sind neu und bilden ebenfalls einen Gegenstand dieser Erfindung.

Die 1,5-Diphenylpyrazol-3-carbonsäurederivate der Formel I können nach verschiedenen, an sich bekannten Synthesewegen hergestellt werden. Nach einer in Ber. 25 3143 (1892) beschriebenen Methode stellt man 3-Aethoxycarbonyl-1,5-diphenyl-pyrazol her, indem man molekulare Mengen von Bromacetophenon und Acetessigsäure-äthylester in Gegenwart von Natriumäthylat zum 2-Acetyl-3-benzoylpropionsäure-äthylester kondensiert, zu welchem man die molekulare Menge Phenyldiazoniumchlorid gibt. Das Kondensat, (3-Benzoyl-2-phenylhydrazinoximpropionsäure-äthylester) cyclisiert in der Wärme zum 3-Aethylcarbonyl-1,5-diphenylpyrazol. Die Reaktionsfolge kann schematisch wie folgt dargestellt werden:

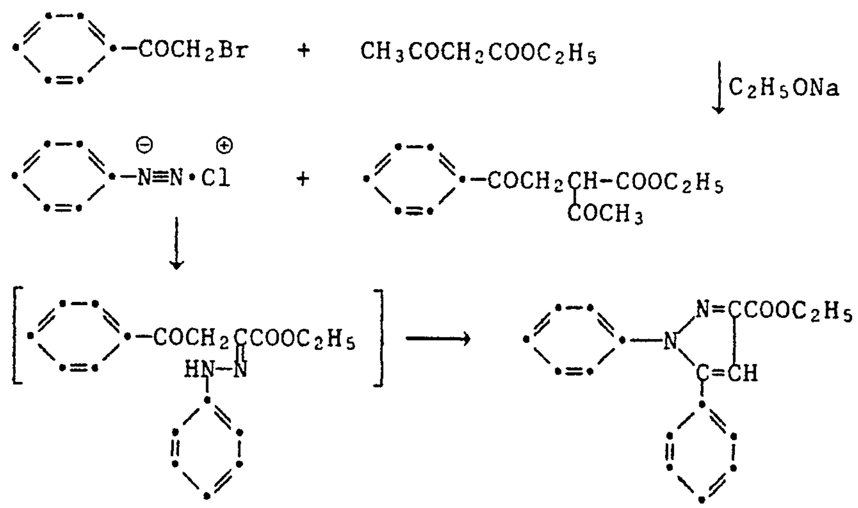


Gemäss einem weiteren in Ber. 47, 1435 (1914) und Can. J. Chem. 41, 1813 (1963) beschriebenen Verfahren kann man die 1,5-Diphenyl-pyrazol-3-carbonsäure und deren Aethylester herstellen, indem man molare Mengen 3-Benzoylpropionsäure und Essigsäureanhydrid zusammengibt, das entstandene Kondensationsprodukt 5-Phenyl-furan-2-carbonyl mit der molaren Menge eines Phenyldiazoniumchlorides versetzt. Das entstandene 2-Phenylhydrazin-5-phenyl-furfuriliden Kondensat kondensiert in der Wärme zum 3-Carbonsäure-1,5-diphenyl-pyrazol oder einem Ester gemäss dem Schema

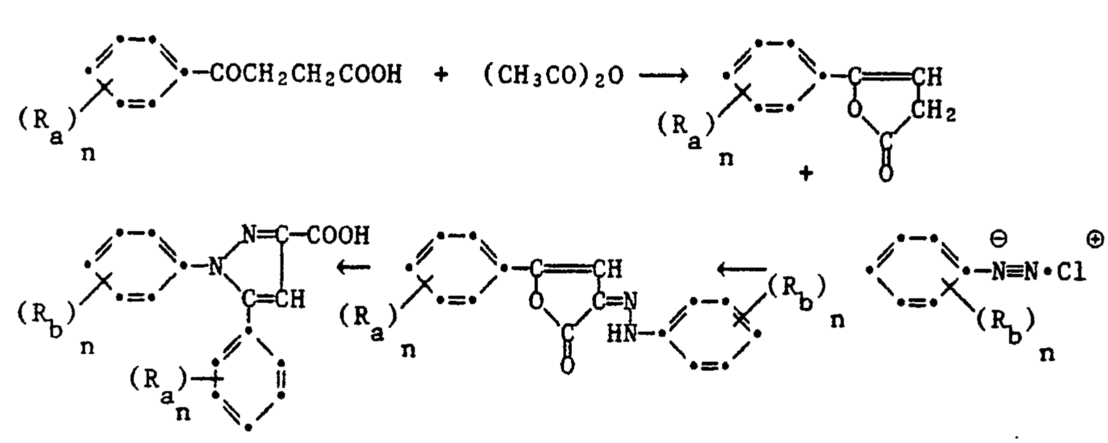

In diesen Formeln haben ($R_a$), ($R_b$) und n die unter Fomel 1 gegebene Bedeutung, $R_b$ bedeutet vorzugsweise ortho Chlor oder para Methyl.

Gemäss einem weiteren Verfahren analog der Ber. 20 2185 (1887) kann man 1,5-Diphenylpyrazol-3-carbonsäuren und deren Ester herstellen indem man in Gegenwart einer Base, z.B. Natrium-Methylat oder Aethylat, äquimolare Mengen von Acetophenon und Oxalsäuredimethylester zum Benzoylbrenztraubensäuremethylester kondensiert, dazu die äquimolare Menge eines Phenylhydrazines gibt und das Kondensat in saurem Milieu ringschliesst, entsprechend dem Reaktionsschema

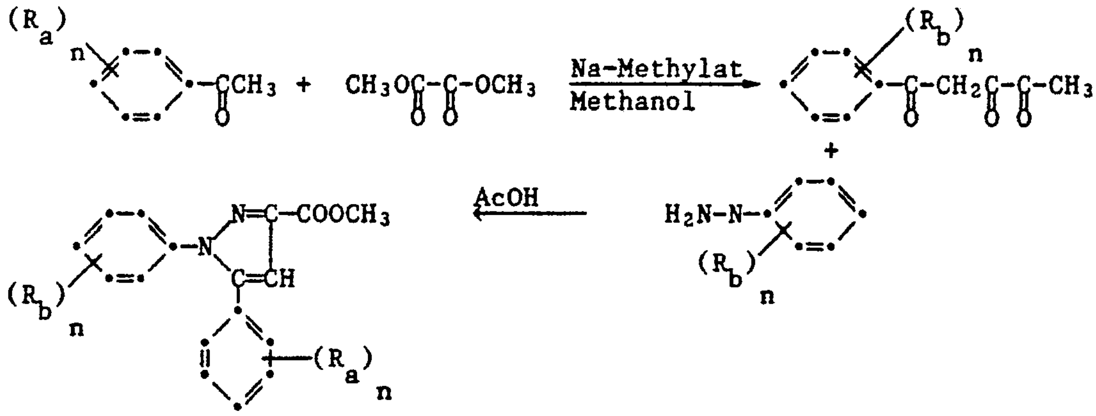


In diesen Formeln haben $R_a$, $R_b$ und n die unter de Formel I gegebene Bedeutung.

Das erfindungsgemässe Verfahren zur Herstellung der 1,5-Diphenylpyrazol-3-carbonsäureester ist dadurch gekennzeichnet, dass man ein Acetophenon der Formel III

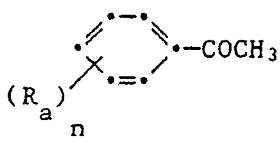
(III)

worin $R_a$ und n die unter der Formel I gegebene Bedeutung haben, in einem inerten organischen Lösungsmittel in Gegenwart einr Base mit der äquimolaren Menge eines Oxalsäurediesters der Formel IV umsetzt

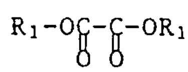
(IV)

worin $R_1$ die unter der Formel I gegebene Bedeutung hat, den entstandenen Benzoylbenztraubensäureester der Formel V

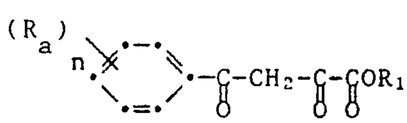
(V)

worin $R_a$, $R_1$ und n die unter der Formel I gegebene Bedeutung haben mit der äquimolaren Menge Phenylhydrazin der Formel VI versetzt

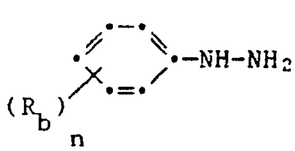
(VI)

worin $R_b$ und n die unter der Formel I gegebene Bedeutung haben, worauf man das Gemisch in saurem Milieu bei erhöhter Temperatur zum 1,5-Diphenylpyrazolcarbonsärederivat der Formel I cyclisiert.

Als inerte Lösungsmittel kommen für die Kondensation des Acetophenons und des Oxalsäureesters die entsprechenden Alkohole wie Methanol, Aethanol oder Aceton höhersiedende Ketone wie Methyläthylketon oder Dioxan, aber auch höhersiedende Aether wie Dipropyläther, Tetrahydrofuran, ferner aromatische Kohlenwasserstoffe wie Benzol, Toluol und Xylol in Betracht.

Die Cyclisation des Kondensates von Benzoylbenztraubensäureester mit dem Hydrazin wird in saurem Milieu vorzugsweise in Eisessig vollzogen.

Die Reaktionstemperaturen liegen zwischen 0°C und 200°C vorzugsweise wird jedoch zwischen 0°C und dem Siedepunkt des Reaktionsgemisches gearbeitet.

Für die Herstellung der Ester der 1,5-Diphenylpyrazol-3-carbonsäuren der Formel I kommen folgende Syntesewege in Frage

1.

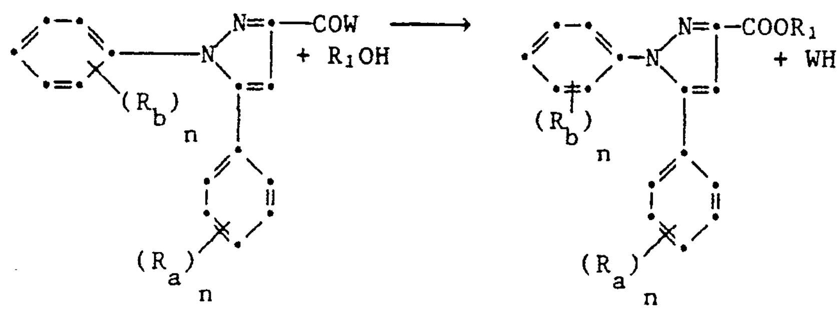

Als Säurederivat W kommt hierbei die unter 1.1 bis 1.5 aufgeführten Reste in Frage.

1.1. Halogen oder der Imidazolrest

1.2. Hydroxy

Diese Umsetzungen verlaufen glatt zum Teil schon bei Raumtemperatur in einem den Reaktionspartnern gegenüber inerten Lösungs- und Verdünnungsmittel. Wenn das Säurehalogenid verwendet wird, setzt man die äquimolare Menge einer Base als säurebindendes Mittel zu.

Wenn W Hydroxy ist, kommt auch ein wasserentziehendes Mittel wie Cyclohexyldiimid, Schwefelsäure oder Kochen am wasserabscheider in Frage.

1.3. $C_1$-$C_4$-Alkoxy.

Die Umsetzung wird als säure- oder basenkatalysierte Umesterung durchgeführt und findet bei erhöhter Temperatur (Raum- bis Siedetemperatur) einen Ueberschuss an $R_1OH$ welcher auch also Lösungsmittel dienen kann und einer katalytischen Menge einer Säure oder Base statt. Als Lösungsmittel kann auch ein Kohlenwasserstoff, wie Benzol, Toluol oder Xylol, Cyclohexan ein Aether wie Diäthyläther, Tetrahydrofuran oder Dioxan verwendet werden.

1.4. O-CO-$C_1$-$C_4$-Alkyl.

Die Umsetzung von Säureanhydriden verläuft wie diejenige der Halogenide zum Teil schon bei Raumtemperatur in einem inerten Lösungs- oder Verdünnungsmittel. Allenfalls setzt man die äquimolare Menge einer Base als säurebindendes Mittel zu.

1.5.

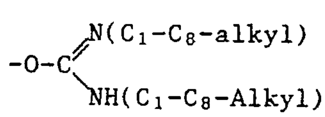

.

Bei dieser Umsetzung spaltet der Amidinrest unter Anlagerung eines Wasserstoffatomes als Harnstoff ab. Diese Veresterung findet in einem inerten wasserfreien Lösungsmittel statt am besten in einem Kohlenwasserstoff wie Benzol, Toluol, Xylol oder Cyclohexan.

2. 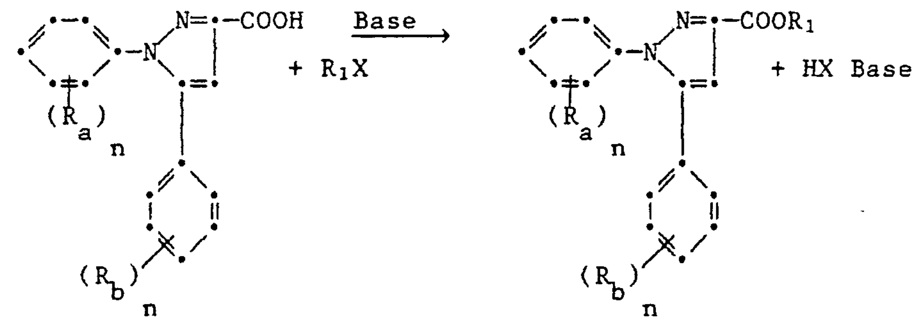


Als X kommt Halogen oder ein Rest - $OSO_2(C_1$-$C_4$-Alkyl) in Frage. Die Umsetzung findet in einem organischen Lösungs- oder Verdünnungsmittel statt, in Gegenwart einer Base wie z.B. einem Alkalimetallalkoholat einem Alkali- oder Erdalkalimetallcarbonat oder -Bicarbonat. Diese Basen sollten gegenüber R-X nicht reaktiv sein.

Im nachfolgenden Beispiel ist die Herstellung eines erfindungsgemässen 1,5-Diphenylpyrazol-3-carbonsäureesters beschrieben. Die Temperaturen sind in Centigraden gegeben.

Beispiele für weitere 1,5-Diphenylpyrazol-3-carbonsäurederivate der Formel I sind in der Tabelle 2 aufgeführt.

Beispiel 1
Herstellung von 1-(2-Chlorphenyl)-3-methoxycarbonyl-5-phenyl-pyrazol

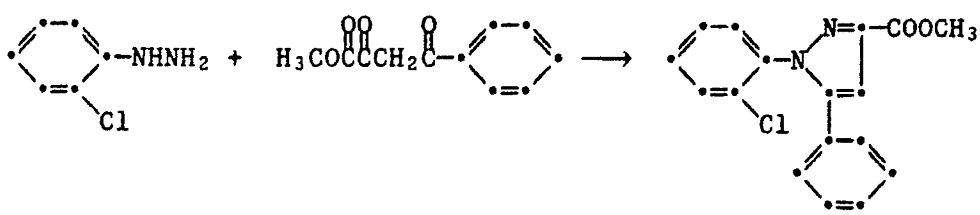

(a) Zu einer Lösung von 21,2 g (0,103 mol) Benzoylbrenztraubensäuremethylester in 100 ml Eisessig gibt man 19,3 g (0,105 mol) 2-Chlorphenylhyrazin-Hydrochlorid (97%ig) und 8,6 g (0,105 mol) Natriumacetat. Nachdem das Reaktionsgemisch 2 Stunden unter Rückfluss gekocht wurde, wird es auf Raumtemperatur gekühlt und anschliessend in Eiswasser gegossen. Das dabei ausfallende Harz wird in Aethylacetat aufgenommen und mit Wasser und 1 M Sodalösung neutral gewaschen. Die organische Phase wird abgetrennt, über Natriumsulfat getrocknet und am Rotationsverdampfer eingeengt. Das zurückbleibende Oel wird aus Diisopropylähter kristallisiert. Man erhält so 17,6 g Titelprodukt vom Schmelzpunkt 67-70°.

Der als Ausgangsprodukt benötigte Benzoylbenztraubensäuremethylester wird wie folgt hergestellt.

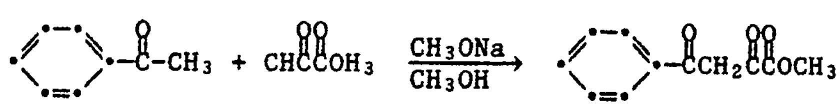


b) Eine Mischung von 75 ml (0,405 mol) 30%ige Natriummethylat-Lösung und 30 ml Methanol wird auf 0° abgekühlt und dann unter Rühren langsam mit 48 g (0.4 mol) Acetophenon versetzt. Zu diesem Gemisch gibt man tropfenweise unter Rühren bei 8-10° eine Lösung von 47,2 g (0,4 mol) Oxalsäuredimethylester in 100 ml Methanol. Das Reaktionsgemisch wird dabei trübe und schliesslich bildet sich ein gelber dicker Niederschlage aus, der sich kaum mehr rühren lässt. Man gibt soviel Methanol zu, bis sich das Reaktionsgemisch wieder rührbar wird. Nach 3 Stunden Weiterrühren bei Raumtemperatur wird abgenutscht, der Niederschlag mit Diäthyläther gewaschen und trocken gesaugt. Das Filtergut wird in Wasser suspendiert und unter Rühren und Kühlen mit Eisessig auf pH 4 gestellt. Der Benzoylbrenztraubensäuremethylester fällt dabeu aus. Er wird abfiltriert, mit Eiswasser gewaschen und im Exsiccator über

Phosphorpentroxid getrocknet. Man erhält 42 g Ester welcher einen Schmelzpunkt von 56-58° hat.

Beispiel 2
Herstellung von 1-(2-Chlorphenyl)-5-phenyl-pyrazol-3-carbonsäurechlorid.

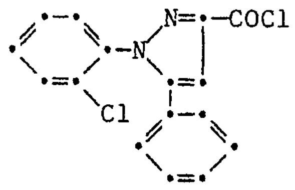

Eine Mischung von 149,4 g 1-(2-Chlorphenyl)-5-phenylpyrazol-3-carbonsäure und 2000 ml trockenes Aethylenchlorid wird auf 70° erwärmt. Dazu tropft man unter Rühren während 12 Minuten 40 ml Thionylchlorid. Das Reaktionsgemisch wird dann während 2½ Stunden bei Rückflusstemperatur gehalten, dann auf Raumtemperatur abgekühlt und am Vakuum eingedampft. Der Rückstand wird aus Cyclohexan-Hexan auskristallisiert. Man erhält so 126 g des obigen Säurechlorids, welches einen Schmelzpunkt von 101 - 102° hat.

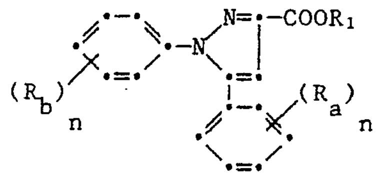

(I)

Tabelle 2

| No. | $(R_a)_n$ | $(R_b)_n$ | $R_1$ | phys. Daten |
|---|---|---|---|---|
| 1 | - | 2-Cl | $CH_3$ | Smp. 67 - 70° |
| 2 | - | 2-Cl | $C_2H_5$ | |
| 3 | - | 2-Cl | $C_3H_7$-n | |
| 4 | - | 2-Cl | $CH(CH_3)_2$ | |
| 5 | - | 2-Cl | $CH_2CH(CH_3)_2$ | |
| 6 | - | 2-Cl | Tetrahydrofurfuryl | |
| 7 | - | 2-Cl | $CH_2CH_2CH(CH_3)_2$ | |
| 8 | - | 2-Cl | $CH_2CH(C_2H_5)_2$ | |
| 9 | - | 2-Cl | $CH(CH_3)CH_2OCH_3$ | |
| 10 | - | 2-Cl | $CH_2CH_2OCH(CH_3)_2$ | |
| 11 | - | 2-Cl | $CH_2CH=CH_2$ | |
| 12 | - | 2-Cl | $CH_2C(CH_3)=CH_2$ | Smp. 66 - 70° |
| 13 | - | 2-Cl | $CH_2CH_2Cl$ | |
| 14 | - | 2-Cl | $CH_2CH(CH_3)CH_2CH_3$ | |
| 15 | - | 2-Cl | $CH_2C\equiv CH$ | Smp. 114 - 115° |
| 16 | - | 2-Cl | $CH_2CH_2C\equiv CH$ | |
| 17 | - | 2-Cl | $C(CH_3)_2CH_2CH_3$ | |
| 18 | - | 2-Cl | $CH(CH_3)COOCH_3$ | |
| 19 | - | 2-Cl | $C(CH_3)_2COOC_2H_5$ | |
| 20 | - | 2-Cl | $CH_2CH_2CN$ | |
| 21 | - | 2-Cl | Pyran-2-ylmethyl | |
| 22 | - | 2-Cl | Benzyl | Smp. 142 - 144° |
| 23 | - | 2-Cl | $CH_2CCl_3$ | |
| 24 | - | 2-Cl | Phenoxyethyl | Smp. 84 - 91° |
| 25 | - | 2-Cl | Cyclopropylmethyl | |
| 26 | - | 2-Cl | Cyclopentylmethyl | |
| 27 | - | 2-Cl | 2,2-Dimethyl-1,3-dioxolan-4-ylmethyl | |

| No. | $(R_a)_n$ | $(R_b)_n$ | $R_1$ | phys. Daten |
|---|---|---|---|---|
| 28 | - | 2-Cl | 2-Thenyl | Smp. 147 - 149° |
| 29 | - | 2-Cl | $CH_2CH=CHCl$ | |
| 30 | - | 2-Cl | $CH_2C(Cl)=CH_2$ | |
| 31 | - | 2-Cl | $CH_2CH_2Si(CH_3)_3$ | Smp. 88 - 89° |
| 32 | - | 2-Cl | $CH_2Si(CH_3)_3$ | |
| 33 | - | 2-Cl | Pyrazol-1-yl-äthyl | |
| 34 | - | 2-Cl | $CH_2CH(NO_2)CH_3$ | |
| 35 | - | 2-Cl | $CH_2CH=CHCH_3$ | |
| 36 | - | 2-Cl | Cyclohexyl | Smp. 144 - 145° |
| 37 | - | 2-Cl | $(CH_2)_9CH_3$ | |
| 38 | - | 2-Cl | $CH_2CH_2N(C_2H_5)_2$ | |
| 39 | - | 2-Cl | $CH_2CH_2OCH_2CH_2OC_2H_5$ | |
| 40 | - | 2-Cl | $CH_2CH(Br)CH_2Br$ | |
| 41 | - | 2-Cl | $CH(CH_2Cl)_2$ | |
| 42 | - | 2-Cl | $CH_2CH_2NO_2$ | |
| 43 | - | 2-Cl | $CH_2C(CH_3)_2N(CH_3)_2$ | |
| 44 | - | 2-Cl | $CH(CH_3)CH_2N(CH_3)_2$ | |
| 45 | - | 2-Cl | 2,6-Dimethylcyclo-hexyl | |
| 46 | - | 2-Cl | 2-Furfuryl | |
| 47 | - | 2-Cl | Morpholinoäthyl | |
| 48 | - | 2-Cl | $CH_2CN$ | |
| 49 | - | 2-Cl | Cyclohexylmethyl | Smp. 103 - 104° |
| 50 | - | 2-Cl | Pyrazol-1-yl-prop-2-yl | Smp. 91 - 92° |
| 51 | - | 2-Cl | $CH_2CH_2SO_2CH_3$ | |
| 52 | - | 2-Cl | $CH_2COOCH_3$ | Smp. 143 - 145° |
| 53 | - | 2-Cl | $CH(CH_3)(CH_2)_4CH_3$ | $n_D^{25}$ 1.5470 |
| 54 | - | 2-Cl | $CH(CH=CH_2)(CH_2)_4CH_3$ | |
| 55 | - | 2,4 $Cl_2$ | $CH_3$ | Smp. 140 - 143° |
| 56 | - | 2,4 $Cl_2$ | $C_2H_5$ | |
| 57 | - | 2,4 $Cl_2$ | $CH(CH_3)_2$ | |
| 58 | - | 2,4 $Cl_2$ | $CH(CH_3)(CH_2)_4CH_3$ | |
| 59 | - | 2,4 $Cl_2$ | $CH_2CH=CH_2$ | |
| 60 | - | 2,4 $Cl_2$ | $CH_2C\equiv CH$ | |

| No. | $(R_a)_n$ | $(R_b)_n$ | $R_1$ | phys. Daten |
|---|---|---|---|---|
| 61 | - | 2,4 $Cl_2$ | Benzyl | |
| 62 | - | 2,4 $Cl_2$ | $(CH_2)_7CH_3$ | |
| 63 | - | 2,3 $Cl_2$ | $CH_3$ | |
| 64 | - | 2,3 $Cl_2$ | $C_2H_5$ | |
| 65 | - | 2,3 $Cl_2$ | $CH(CH_3)_2$ | |
| 66 | - | 2,3 $Cl_2$ | $CH(CH_3)(CH_2)_4CH_3$ | |
| 67 | - | 2,3 $Cl_2$ | $CH_2CH{=}CH_2$ | |
| 68 | - | 2,3 $Cl_2$ | $CH_2C{\equiv}CH$ | |
| 69 | - | 2,3 $Cl_2$ | Benzyl<br>$(CH_2)_7CH_3$ | |
| 70 | - | 2-F | $CH_3$ | Smp. 83 - 85° |
| 71 | - | 2-F | $C_2H_5$ | |
| 72 | - | 2-F | $CH(CH_3)_2$ | |
| 73 | - | 2-F | $CH(CH_3)(CH_2)_4CH_3$ | |
| 74 | - | 2-F | $CH_2CH{=}CH_2$ | |
| 75 | - | 2-F | $CH_2CH{\equiv}CH$ | |
| 76 | - | 2-F | Benzyl | |
| 77 | - | 2-F | $(CH_2)_7CH_3$ | |
| 78 | - | 2-$OCH_3$ | $CH_3$ | Smp. 126 - 128° |
| 79 | - | 2-$OCH_3$ | $C_2H_5$ | |
| 80 | - | 2-$OCH_3$ | $CH(CH_3)_2$ | |
| 81 | - | 2-$OCH_3$ | $CH(CH_3)(CH_2)_4CH_3$ | |
| 82 | - | 2-$OCH_3$ | $CH_2CH{=}CH_2$ | |
| 83 | - | 2-$OCH_3$ | $CH_2C{\equiv}CH$ | |
| 84 | - | 2-$OCH_3$ | Benzyl | |
| 85 | - | 2-$OCH_3$ | $(CH_2)_7CH_3$ | |
| 86 | - | 2,5 $Cl_2$ | $CH_3$ | |
| 87 | - | 2,5 $Cl_2$ | $C_2H_5$ | |
| 88 | - | 2,5 $Cl_2$ | $CH(CH_3)_2$ | |
| 89 | - | 2,5 $Cl_2$ | $CH(CH_3)(CH_2)_4CH_3$ | |
| 90 | - | 2,5 $Cl_2$ | $CH_2CH{=}CH_2$ | |
| 91 | - | 2,5 $Cl_2$ | $CH_2C{\equiv}CH$ | |
| 92 | - | 2,5 $Cl_2$ | Benzyl | |
| 93 | - | 2,5 $Cl_2$ | $(CH_2)_7CH_3$ | |
| 94 | - | 2-Cl, 4-$CH_3$ | $CH_3$ | |

| No. | $(R_a)_n$ | $(R_b)_n$ | $R_1$ | phys. Daten |
|---|---|---|---|---|
| 95 | - | 2-Cl, 4-$CH_3$ | $C_2H_5$ | |
| 96 | - | 2-Cl, 4-$CH_3$ | $CH(CH_3)_2$ | |
| 97 | - | 2-Cl, 4-$CH_3$ | $CH(CH_3)(CH_2)_4CH_3$ | |
| 98 | - | 2-Cl, 4-$CH_3$ | $CH_2CH{=}CH_2$ | |
| 99 | - | 2-Cl, 4-$CH_3$ | $CH_2C{\equiv}CH$ | |
| 100 | - | 2-Cl, 4-$CH_3$ | Benzyl | |
| 101 | - | 2-Cl, 4-$CH_3$ | $(CH_2)_7CH_3$ | |
| 102 | - | 2-Br | $CH_3$ | Smp. 81 - 83° |
| 103 | - | 2-Br | $C_2H_5$ | |
| 104 | - | 2-Br | $CH(CH_3)_2$ | |
| 105 | - | 2-Br | $CH(CH_3)(CH_2)_4CH_3$ | |
| 106 | - | 2-Br | $CH_2CH{=}CH_2$ | |
| 107 | - | 2-Br | $CH_2C{\equiv}CH$ | |
| 108 | - | 2-Br | Benzyl | |
| 109 | - | 2-Br | $(CH_2)_7CH_3$ | |
| 110 | - | 2-$CH_3$ | $CH_3$ | Smp. 105 - 106° |
| 111 | - | 2-$CH_3$ | $C_2H_5$ | |
| 112 | - | 2-$CH_3$ | $CH(CH_3)_2$ | |
| 113 | - | 2-$CH_3$ | $CH(CH_3)(CH_2)_7CH_3$ | |
| 114 | - | 2-$CH_3$ | $CH_2CH{=}CH_2$ | |
| 115 | | 2-$CH_3$ | $CH_2C{\equiv}CH$ | |
| 116 | - | 2-$CH_3$ | Benzyl | |
| 117 | - | 2-$CH_3$ | $(CH_2)_7CH_3$ | |
| 118 | - | 2,4 $Cl_2$, 5-$OCH_3$ | $CH_3$ | |
| 119 | - | 2,4 $Cl_2$, 5-$OCH_3$ | $C_2H_5$ | |
| 120 | - | 2,4 $Cl_2$, 5-$OCH_3$ | $CH(CH_3)_2$ | |
| 121 | - | 2,4 $Cl_2$, 5-$OCH_3$ | $CH(CH_3)(CH_2)_4CH_3$ | |
| 122 | - | 2,4 $Cl_2$, 5-$OCH_3$ | $CH_2CH{=}CH_2$ | |
| 123 | - | 2,4 $Cl_2$, 5-$OCH_3$ | $CH_2C{\equiv}CH$ | |
| 124 | - | 2,4 $Cl_2$, 5-$OCH_3$ | Benzyl | |
| 125 | - | 2,4 $Cl_2$, 5-$OCH_3$ | $(CH_2)_7CH_3$ | |
| 126 | 3-$CH_3$ | 2-Cl | $CH_3$ | Smp. 129 - 130° |
| 127 | 3-Cl | 2-Cl | $CH_3$ | Smp. 141 - 143° |
| 128 | 3-$OCH_3$ | 2-Cl | $CH_3$ | Smp. 115 - 117° |

| No. | $(R_a)_n$ | $(R_b)_n$ | $R_1$ | phys. Daten |
|---|---|---|---|---|
| 129 | 3-F | 2-Cl | $CH_3$ | Smp. 114 - 116° |
| 130 | - | - | $CH_3$ | Smp. 84 - 85° |
| 131 | - | $2-NO_2$ | $CH_3$ | Smp. 208 - 210° |
| 132 | - | - | $C_2H_5$ | Smp. 85 - 87° |
| 133 | - | 2-Cl | $(CH_2CH=C(CH_3)HCH_2)_2CH_2CH(CH_3)_2$ | |
| 134 | - | 2-Cl | $-CH_2(CH_2)_3CH=CH_2$ | $n_D^{20}$ 1.5818 |
| 135 | - | 2-Cl | 2-Chlorbenzyl | Smp. 96 - 98° |
| 136 | - | 2-Cl | $-(CH_2)_7CH_3$ | $n_D^{20}$ 1.5532 |
| 137 | - | 2-Cl | $-(CH_2)_{11}-CH_3$ | Smp. 66 - 67° |
| 138 | - | 2-Cl | 5-Chlor-2-methoxy-phenyl-äthyl | Smp. 129 - 130° |
| 140 | - | 2-Cl | Pyridin-3-yl | Smp. 110 - 111° |
| 141 | - | 2-Cl | Pyridin-2-yl | |
| 142 | - | 2-Cl | Tetrahydrofuryl-3 | Smp. 95 - 97° |
| 143 | - | 2-Cl | 5-Methyl-thiazol-4-yläthyl | Smp. 90 - 91° |
| 144 | - | 2-Cl | Thiophen-2-yl-ethyl | Smp. 123 - 124° |
| 145 | - | 2-Cl | $-(CH_2)_3CH_3$ | Smp. 66 - 68° |
| 146 | - | 2-Cl | $-CH_2-CONH_2$ | Smp. 192 - 194° |
| 147 | - | 2-Cl | $-(CH_2)_8CH=CH(CH_2)_7CH_3$ | |
| 148 | - | 2-Cl | $HN^{\oplus}(C_2H_5)_3$ | Smp. 163 - 166° |
| 149 | - | 2-Cl | $H_3N^{\oplus}CH(CH_3)_2$ | Smp. 165 - 168° |
| 150 | - | 2-Cl | 2,3.4,6.7.8.9.-10-Octahydropyrimido-[2,1-a]-azepin-1-ium | Smp. 204 - 206° |
| 151 | - | 2-Cl | $Na^{\oplus}$ | Smp. > 260° |
| 152 | - | 2-Cl | H | Smp. 198 - 200° |
| 153 | - | 2-Cl | $OR_1$ = Cl (Säurechlorid) | Smp. 101 - 102° |
| 154 | - | 2-F | $OR_1$ = Cl (Säurechlorid) | |
| 155 | 3-F | 2-Cl | $OR_1$ = Cl (Säurechlorid) | |
| 156 | 2-F | - | $OR_1$ = Cl (Säurechlorid) | |
| 157 | 2-Cl | - | $OR_1$ = Cl (Säurechlorid) | |
| 158 | - | $2-CH_3$ | $OR_1$ = Cl (Säurechlorid) | |
| 159 | 2-Cl | 2,4 $(Cl_2)$ | $CH_3$ | Smp. 84 - 88° |

| No. | $(R_a)_n$ | $(R_b)_n$ | $R_1$ | phys. Daten |
|---|---|---|---|---|
| 160 | 2-Cl | 2-F | $CH_3$ | Smp. 84 - 87° |
| 161 | 2-F | $3-OCH_3$ | $CH_3$ | Smp. 114 - 115° |
| 162 | - | $3-OCH_3$ | $CH_3$ | Smp. 103 - 106° |
| 163 | 2F, 4Cl $SOC_3H_7iso$ | $3-OCH_3$ | $CH_3$ | Smp. 82 - 84° |
| 164 | $2,4-Cl_2$ | $3-OCH_3$ | $CH_3$ | Smp. 109 - 111° |
| 165 | - | 2-Cl | $-(CH_2)_{17}-CH_3$ | Smp. 77 - 79° |
| 166 | - | 2-Cl | $-(CH_2CH{=}C(CH_3)CH_2-)_3H$ | $n_D^{20}$ 1.5580 |
| 167 | - | 2-Cl | $-CH(CH_2)_7CH{=}CH(CH_2)_7CH_3$ | $n_D^{22}$ 1.5375 |

Die erfindungsgemässen 1,5-Diphenylpyrazol-3-carbonsäurederivate der Formel I werden als Safener in Mischung mit 2-[4-(Phenyl, Pyridin-2-yl-, 4-Benzoxazol-, 4-Benzthiazol- und 4-Chinoxalin-2-yl)oxyphenoxy]-propionsäureester-Herbiziden der Formel II zur Bekämpfung von Unkräutern in Nutzpflanzenkulturen verwendet.

Bei den zu bekämpfenden Unkräutern kann es sich sowohl um monokotyle wie um dikotyle Unkräuter handeln.

Als Kulturpflanzen oder Teile dieser Pflanzen kommen beispielsweise die vorstehen genannten in Betracht. Als Anbauflächen gelten die bereits mit den Kulturpflanzen bewachsenen oder mit dem Saatgut dieser Kulturpflanzen beschickten Bodenareale, wie auch die zur Bebauung mit diesen Kulturpflanzen bestimmten Böden.

Die zu applizierende Aufwandmenge Antidot im Verhältnis zum Herbizid richtet sich weitgehend nach der Anwendungsart. Bei einer Feldbehandlung, welche entweder unter Verwendung einer Tankmischung mit einer Kombination von Antidot und Herbizid oder durch getrennte Applikation von Antidot und Herbizid erfolgt, liegt in der Regel ein Verhältnis von Antidot zu Herbizid von 1:100 bis 10:1, bevorzugt 1:1 bis 10:1, und insbesondere 1:1, vor. Dagegen werden bei der Samenbeizung weit geringer Mengen Antidot im Verhältnis zur Aufwandmenge an Herbizid pro Hektar Anbaufläche benötigt.

In der Regel werden bei der Feldbenhandlung 0,001 bis 1 kg Antidot/ha, vorzugsweise 0,02 bis 0,2 kg Antidot/ha, appliziert,

Bei der Samenbeizung werden im allgemeinen 0,01 bis 10 g Antidot/kg Samen, vorzugsweise 0,05 bis 2 g Antidot/kg Samen, appliziert. Wird das Antidot in flüssiger Form kurz vor der Aussaat unter Samenquellung appliziert, so werden zweckmässigerweise Antidot-Lösungen verwendet, welche den Wirkstoff in einer Konzentration von 1 bis 10 000, vorzugsweise von 10 bis 1 000 ppm, enthalten.

Zur Applikation werden die Verbindungen der Formel I oder Kombinationen von Verbindungen der Formel I mit den zu antagonisierenden Herbiziden zweckmässigerweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen, Bestreichen oder Giessen werden gleich wie die Art der zu verwendenden Mittel den angestrebten Zielen und den gegebenen Verhältnissen entsprechend gewählt.

Die Formulierungen, d.h. die den Wirkstoff der Formel I oder eine Kombination von Wirkstoff der Formel I mit zu antagonisierendem Herbizid und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmiteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie

deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffs der Formel I und gegebenenfalls auch dem zu antagonisierenden Herbizid nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche also N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood New Jersey, 1981.
Stache, H., "Tensid-Taschenbuch",

Carl Hanser Verlag, München/Wien, 1981.

Die agrochemischen Zubereitungen enthalten in der Regel 0,1 bis 99 Gewichtsprozent, insbesondere 0,1 bis 95 Gew.-%, Wirkstoff der Formel I oder Wirkstoffgemisch Antidot/Herbizid, 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Für die Verwendung von Verbindungen der Formel I oder sie enthaltender Mittel zum Schützen von Kulturpflanzen gegen schädigende Wirkungen von Herbiziden der Formel II kommen verschiedene Methoden und Techniken in Betracht, wie beispielsweise die folgenden:

i) Samenbeizung

a) Beizung der Samen mit einem als Spritzpulver formulierten Wirkstoff der Formel I durch Schütteln in einem Gefäss bis zur gleichmässigen Verteilung auf der Samenoberfläche (Trockenbeizung). Man verwendet dabei etwa 10 bis 500 g Wirkstoff der Formel I (40 g bis 2 kg Spritzpulver) pro 100 kg Saatgut.

b) Beizung der Samen mit einem Emulsionskonzentrat des Wirksoffs der Formel I nach der Methode a) (Nassbeizung).

c) Beizung durch Tauchen des Saatguts in eine Brühe mit 10-1000 ppm Wirkstoff der Formel I während 1 bis 72 Stunden und gegebenenfalls nachfolgendes Trocknen der Samen (Tauchbeizung).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 10 bis 500 g Antidot, vorzugsweise 20 bis 250 g Antidot, pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Antidot und Herbizid (gegenseitiges Mengenverhältnis zwischen 100:1 und 1:10) wird verwendet, wobei die Aufwandmenge an Herbizid 0,001 bis 10 kg pro Hektar beträgt. Solche Tankmischung wird vor oder nach der Aussaat appliziert.

iii) Applikation in der Saatfurche

Das Antidot wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff der Formel I wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Formulierungsbeispiel für flüssige Wirkstoffe der Formel I (% = Gewichtsprozent)

| 1. Emulsions-Konzentrate | a) | b) | c) |
|---|---|---|---|
| Wirkstoff aus Tabelle 2 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff aus Tabelle 2 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°) | - | - | 94 % | - |

Die Lösungen sind zur Anwendung in Form kleinster Tropfen geeignet.

| 3. Granulate | a) | b) |
|---|---|---|
| Wirkstoff aus Tabelle 2 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff aus Tabelle 2 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemitel.

Formulierungsbeispiele für feste Wirkstoffe der Formel I (% = Gewichtsprozent)

| 5. Spritzpulver | a) | b) | c) |
|---|---|---|---|
| Wirkstoff aus Tabelle 2 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen.

Man erhält Spritzpulver, die sich mit Wasser zu Suspensionen jeder gewünschten Konzentration verdünnen lassen.

| 6. Emulsions-Konzentrate | |
|---|---|
| Wirkstoff aus Tabelle 2 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff aus Tabelle 2 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 8. Extruder-Granulate | |
|---|---|
| Wirkstoff aus Tabelle 2 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 9. Umhüllungs-Granulate | |
|---|---|
| Wirkstoff aus Tabelle 2 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 10. Suspensions-Konzentrate | |
|---|---|
| Wirkstoff aus Tabelle 2 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten

Konzentration hergestellt werden können.

Formulierungsbeispiele für Wirkstoffgemische (flüssig (% = Gewichtsprozent)

| 11. Emulsions-Konzentrate | a) | b) | c) |
|---|---|---|---|
| Wirkstoffgemisch:Antidot aus Tabelle 2 und ein Herbizid der Tabelle 1 im Verhältnis 1:1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 12. Emulsions-Konzentrate | a) | b) | c) |
|---|---|---|---|
| Wirkstoffgemisch: Antidot aus Tabelle 2 und ein Herbizid der Tabelle 1 im Verhältnis 1:3 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 13. Emulsions-Konzentrate | a) | b) | c) |
|---|---|---|---|
| Wirkstoffgemisch: Antidot aus Tabelle 2 und ein Herbizid der Tabelle 1 im Verhältnis 2:1 | 25 % | 40 % | 50 % |
| Ca-Dodecylbenzolsulfonat | 5 % | 8 % | 6 % |
| Ricinusöl-polyäthylenglykoläther (36 Mol AeO) | 5% | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 14. Emulsions-Konzentrate | a) | b) | c) |
|---|---|---|---|
| Wirkstoffgemisch: Antidot aus Tabelle 2 und 2-[4-(5-Chlor-3-fluorpyridin-2-yloxy)-phenoxy]-propionsäure-methylester im Verhältnis 1:1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 15. Emulsions-Konzentrat | a) | b) | c) |
|---|---|---|---|
| Wirkstoffgemisch: Antidot aus Tabelle 2 und 2-[4-(5-Chlor-3-fluorpyridin-2-yloxy)-phenoxy]-propionsäure-methylester im Verhältnis 1:3 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 16. Lösungen | a) | b) | c) | d) |
|---|---|---|---|---|
| Wirkstoffgemisch: Antidot aus Tabelle 2 und ein Herbizid der Tabelle 1 im Verhältnis 1:4 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190° C) | - | - | 94 % | - |

Die Lösungen sind zur Anwendung in Form kleinster Tropfen geeignet.

| 17. Lösungen | a) | b) | c) | d) |
|---|---|---|---|---|
| Wirkstoffgemisch: Antidot aus Tabelle 2 und ein Herbizid aus Tabelle 1 im Verhältnis 5:2 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190 °C) | - | - | 94 % | - |

Die Lösungen sind zur Anwendung in Form kleinster Tropfen geeignet.

| 18. Lösungen | a) | b) | c) | d) |
|---|---|---|---|---|
| Wirkstoffgemisch: Antidot aus Tabelle 2 und ein Herbizid aus Tabelle 1 im Verhältnis 1:1 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190 °C) | - | - | 94 % | - |

Die Lösungen sind zur Anwendung in Form kleinster Tropfen geeignet.

| 19. Lösungen | a) | b) | c) | d) |
|---|---|---|---|---|
| Wirkstoffgemisch: Antidot aus Tabelle 2 und 2-[4-(5-Chlor-3-fluorpyridin-2-yloxy)-phenoxy]-propionsäure-methylester im Verhältnis 1:1 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 20. Lösungen | a) | b) | c) | d) |
| --- | --- | --- | --- | --- |
| Wirkstoffgemisch: Antidot aus Tabelle 2 und 2-[4-(5-Chlor-3-fluorpyridin-2-yloxy)-phenoxy]-propionsäure-methylester im Verhältnis 1:4 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 21. Granulate | a) | b) |
| --- | --- | --- |
| Wirkstoffgemisch: Antidot aus Tabelle 2 und ein Herbizid der Tabelle 1 im Verhältnis 1:1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 22. Granulate | a) | b) |
| --- | --- | --- |
| Wirkstoffgemisch: Antidot aus Tabelle 2 und 2-[4-(5-Chlor-3-fluorpyridin-2-yloxy)-phenoxy]-propionsäure-methylester im Verhältnis 1:1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 23. Stäubemittel | a) | b) |
| --- | --- | --- |
| Wirkstoffgemisch: Antidot aus Tabelle 2 und ein Herbizid der Tabelle 2 im Verhältnis 1:1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für Wirkstoffgemische (fest) (% = Gewichtsprozent)

| 24. Spritzpulver | a) | b) | c) |
|---|---|---|---|
| Wirkstoffgemisch: Antidot aus Tabelle 2 und ein Herbizid der Tabelle 1 im Verhältnis 1:1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 25. Spritzpulver | a) | b) | c) |
|---|---|---|---|
| Wirkstoffgemisch: Antidot aus Tabelle 2 und ein Herbizid der Tabelle 1 im Verhältnis 1:4 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser Zu Suspensionen jeder gewünschten Konzentration verdünnen lassen.

| 26. Spritzpulver | a) | b) | c) |
|---|---|---|---|
| Wirkstoffgemisch: Antidot aus Tabelle 2 und ein Herbizid der Tabelle 1 im Verhältnis 3:1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 27. Emulsions-Konzentrate | |
|---|---|
| Wirkstoffgemisch: Antidot aus Tabelle 2 und ein Herbizid der Tabelle 1 im Verhältnis 1:1 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 28. Emulsions-Konzentrate | |
|---|---|
| Wirkstoffgemisch: Antidot aus Tabelle 2 und ein Herbizid der Tabelle 1 im Verhältnis 5:2 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 29. Emulsions-Konzentrate | |
|---|---|
| Wirkstoffgemisch: Antidot aus Tabelle 2 und ein Herbizid der Tabelle 1 im Verhältnis 1:4 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 30. Stäubemittel | a) | b) |
|---|---|---|
| Wirkstoffgemisch: Antidot aus Tabelle 2 und ein Herbizid der Tabelle 1 im Verhältnis 1:1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 31. Extruder-Granulate | |
|---|---|
| Wirkstoffgemisch: Antidot aus Tabelle 2 und ein Herbizid der Tabelle 1 im Verhältnis 1:1 | 10 % |
| Na-Liginsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 32. Umhüllungs-Granulate | |
|---|---|
| Wirkstoffgemisch: Antidot aus Tabelle 2 und ein Herbizid der Tabelle 1 im Verhältnis 1:1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 33. Suspensions-Konzentrate | |
|---|---|
| Wirkstoffgemisch: Antidot aus Tabelle 2 und ein Herbizid der Tabelle 1 im Verhältnis 1:1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| 34. Suspensions-Konzentrate | |
|---|---|
| Wirkstoffgemisch: Antidot aus Tabelle 2 und ein Herbizid der Tabelle 1 im Verhältnis 1:4 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| 35. Suspensions-Konzentrate | |
|---|---|
| Wirkstoffgemisch: Antidot aus Tabelle 2 und ein Herbizid der Tabelle 1 im Verhältnis 3:1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

<u>Biologisches Beispiel</u>

Testbeschreibung

Im Gewächshaus werden Plastiktöpfe, welche 0,5 l Erde enthalten, mit Samen der zu testenden Pflanzen beschickt. Wenn die Pflanzen das 2-bis 3-Blattstadium erreicht haben, werden ein Safener der Formel I und ein Herbizid der Formel II zusammen als Tankmischung appliziert. 21 Tage nach der Applikation wird der Zustand der Pflanzen bonitiert und die Schutzwirkung des Safeners errechnet. Es ist die Differenz des Schadens den das Herbizid an einer Pflnze anrichtet die nicht mit dem Safener behandelt ist und einer die mit Safener behandelt wurde. Die Schutzwirkung wird in Prozent angegeben.

Resultate

Kultur: Sommerweizen "Besso"

Herbizid:

2(R)-2-[4-(5-Chlor-3-fluorpyridin-2-yloxy)phenoxy]-propionsäure-propargylester (Tabelle 1, 2.19 2R-Enantiomer)

| Herbizid Aufwandmenge | Antidot (Tabelle 2) No | Aufwandmenge | Schutzwirkung |
|---|---|---|---|
| 400 g/hg | 001 | 400 g/ha | 75 % |
| | | 200 g/ha | 75 % |
| | | 100 g/ha | 75 % |
| | | 50 g/ha | 75 % |
| 200 g/hg | 001 | 400 g/ha | 63 % |
| | | 200 g/ha | 75 % |
| | | 100 g/ha | 63 % |
| | | 50 g/ha | 75 % |
| 100 g/hg | 001 | 400 g/ha | 50 % |
| | | 200 g/ha | 63 % |
| | | 100 g/ha | 63 % |
| | | 50 g/ha | 63 % |
| 400 g/hg | 012 | 400 g/ha | 88 % |
| | | 200 g/ha | 75 % |
| | | 100 g/ha | 75 % |
| | | 50 g/ha | 75 % |
| 200 g/hg | 012 | 400 g/ha | 75 % |
| | | 200 g/ha | 75 % |
| | | 100 g/ha | 63 % |
| | | 50 g/ha | 63 % |
| 100 g/hg | 012 | 400 g/ha | 50 % |
| | | 200 g/ha | 63 % |
| | | 100 g/ha | 63 % |
| | | 50 g/ha | 63 % |
| 400 g/hg | 024 | 400 g/ha | 88 % |
| | | 200 g/ha | 88 % |
| | | 100 g/ha | 88 % |
| | | 50 g/ha | 75 % |

| Herbizid Aufwandmenge | Antidot (Tabelle 2) No | Aufwandmenge | Schutzwirkung |
|---|---|---|---|
| 200 g/hg | 024 | 400 g/ha | 75 % |
| | | 200 g/ha | 75 % |
| | | 100 g/ha | 75 % |
| | | 50 g/ha | 75 % |
| 100 g/hg | 024 | 400 g/ha | 63 % |
| | | 200 g/ha | 63 % |
| | | 100 g/ha | 63 % |
| | | 50 g/ha | 63 % |
| 400 g/hg | 036 | 400 g/ha | 50 % |
| | | 200 g/ha | 50 % |
| | | 100 g/ha | 50 % |
| | | 50 g/ha | 38 % |
| 200 g/hg | 036 | 400 g/ha | 63 % |
| | | 200 g/ha | 75 % |
| | | 100 g/ha | 75 % |
| | | 50 g/ha | 50 % |
| 100 g/hg | 036 | 400 g/ha | 50 % |
| | | 200 g/ha | 50 % |
| | | 100 g/ha | 63 % |
| | | 50 g/ha | 50 % |
| 400 g/hg | 049 | 400 g/ha | 63 % |
| | | 200 g/ha | 63 % |
| | | 100 g/ha | 63 % |
| | | 50 g/ha | 38 % |
| 200 g/hg | 049 | 400 g/ha | 75 % |
| | | 200 g/ha | 88 % |
| | | 100 g/ha | 75 % |
| | | 50 g/ha | 75 % |
| 100 g/hg | 049 | 400 g/ha | 50 % |
| | | 200 g/ha | 63 % |
| | | 100 g/ha | 63 % |
| | | 50 g/ha | 50 % |
| 400 g/hg | 050 | 400 g/ha | 63 % |
| | | 200 g/ha | 63 % |
| | | 100 g/ha | 50 % |
| | | 50 g/ha | 50 % |
| 200 g/hg | 050 | 400 g/ha | 63 % |
| | | 200 g/ha | 75 % |
| | | 100 g/ha | 75 % |
| | | 50 g/ha | 63 % |

| Herbizid Aufwandmenge | Antidot (Tabelle 2) No | Aufwandmenge | Schutzwirkung |
|---|---|---|---|
| 100 g/hg | 050 | 400 g/ha | 50 % |
| | | 200 g/ha | 50 % |
| | | 100 g/ha | 63 % |
| | | 50 g/ha | 38 % |
| 400 g/hg | 052 | 400 g/ha | 88 % |
| | | 200 g/ha | 75 % |
| | | 100 g/ha | 75 % |
| | | 50 g/ha | 88 % |
| 200 g/hg | 052 | 400 g/ha | 75 % |
| | | 200 g/ha | 75 % |
| | | 100 g/ha | 75 % |
| | | 50 g/ha | 75 % |
| 100 g/hg | 052 | 400 g/ha | 63 % |
| | | 200 g/ha | 63 % |
| | | 100 g/ha | 63 % |
| | | 50 g/ha | 63 % |
| 400 g/hg | 055 | 400 g/ha | 50 % |
| | | 200 g/ha | 50 % |
| | | 100 g/ha | 50 % |
| | | 50 g/ha | 50 % |
| 200 g/hg | 055 | 400 g/ha | 63 % |
| | | 200 g/ha | 63 % |
| | | 100 g/ha | 75 % |
| | | 50 g/ha | 63 % |
| 100 g/hg | 055 | 400 g/ha | 63 % |
| | | 200 g/ha | 63 % |
| | | 100 g/ha | 63 % |
| | | 50 g/ha | 63 % |
| 400 g/hg | 070 | 400 g/ha | 63 % |
| | | 200 g/ha | 63 % |
| | | 100 g/ha | 50 % |
| | | 50 g/ha | 80 % |
| 200 g/hg | 070 | 400 g/ha | 63 % |
| | | 200 g/ha | 75 % |
| | | 100 g/ha | 75 % |
| | | 50 g/ha | 63 % |
| 100 g/hg | 070 | 400 g/ha | 50 % |
| | | 200 g/ha | 63 % |
| | | 100 g/ha | 50 % |
| | | 50 g/ha | 63 % |

| Herbizid Aufwandmenge | Antidot (Tabelle 2) No | Aufwandmenge | Schutzwirkung |
|---|---|---|---|
| 400 g/hg | 102 | 400 g/ha | 63 % |
| | | 200 g/ha | 75 % |
| | | 100 g/ha | 63 % |
| | | 50 g/ha | 50 % |
| 200 g/hg | 102 | 400 g/ha | 63 % |
| | | 200 g/ha | 63 % |
| | | 100 g/ha | 75 % |
| | | 50 g/ha | 75 % |
| 100 g/hg | 102 | 400 g/ha | 50 % |
| | | 200 g/ha | 50 % |
| | | 100 g/ha | 50 % |
| | | 50 g/ha | 50 % |
| 400 g/hg | 129 | 400 g/ha | 63 % |
| | | 200 g/ha | 63 % |
| | | 100 g/ha | 63 % |
| | | 50 g/ha | 50 % |
| 200 g/hg | 129 | 400 g/ha | 63 % |
| | | 200 g/ha | 63 % |
| | | 100 g/ha | 75 % |
| | | 50 g/ha | 50 % |
| 100 g/hg | 129 | 400 g/ha | 50 % |
| | | 200 g/ha | 50 % |
| | | 100 g/ha | 50 % |
| | | 50 g/ha | 50 % |
| 400 g/hg | 132 | 400 g/ha | 75 % |
| | | 200 g/ha | 63 % |
| | | 100 g/ha | 50 % |
| | | 50 g/ha | 38 % |
| 200 g/hg | 132 | 400 g/ha | 63 % |
| | | 200 g/ha | 75 % |
| | | 100 g/ha | 63 % |
| | | 50 g/ha | 63 % |
| 100 g/hg | 132 | 400 g/ha | 63 % |
| | | 200 g/ha | 63 % |
| | | 100 g/ha | 63 % |
| | | 50 g/ha | 63 % |
| 400 g/hg | 134 | 400 g/ha | 75 % |
| | | 200 g/ha | 63 % |
| | | 100 g/ha | 63 % |
| | | 50 g/ha | 38 % |

| Herbizid Aufwandmenge | Antidot (Tabelle 2) No | Aufwandmenge | Schutzwirkung |
|---|---|---|---|
| 200 g/hg | 134 | 400 g/ha | 75 % |
| | | 200 g/ha | 75 % |
| | | 100 g/ha | 75 % |
| | | 50 g/ha | 63 % |
| 100 g/hg | 134 | 400 g/ha | 50 % |
| | | 200 g/ha | 63 % |
| | | 100 g/ha | 50 % |
| | | 50 g/ha | 50 % |
| 400 g/hg | 135 | 400 g/ha | 88 % |
| | | 200 g/ha | 88 % |
| | | 100 g/ha | 88 % |
| | | 50 g/ha | 88 % |
| 200 g/hg | 135 | 400 g/ha | 75 % |
| | | 200 g/ha | 75 % |
| | | 100 g/ha | 75 % |
| | | 50 g/ha | 75 % |
| 100 g/hg | 135 | 400 g/ha | 63 % |
| | | 200 g/ha | 63 % |
| | | 100 g/ha | 63 % |
| | | 50 g/ha | 63 % |
| 400 g/hg | 136 | 400 g/ha | 88 % |
| | | 200 g/ha | 88 % |
| | | 100 g/ha | 88 % |
| | | 50 g/ha | 88 % |
| 200 g/hg | 136 | 400 g/ha | 75 % |
| | | 200 g/ha | 75 % |
| | | 100 g/ha | 75 % |
| | | 50 g/ha | 75 % |
| 100 g/hg | 136 | 400 g/ha | 63 % |
| | | 200 g/ha | 63 % |
| | | 100 g/ha | 63 % |
| | | 50 g/ha | 63 % |
| 400 g/hg | 140 | 400 g/ha | 63 % |
| | | 200 g/ha | 63 % |
| | | 100 g/ha | 38 % |
| | | 50 g/ha | 25 % |
| 200 g/hg | 140 | 400 g/ha | 63 % |
| | | 200 g/ha | 63 % |
| | | 100 g/ha | 63 % |
| | | 50 g/ha | 63 % |

| Herbizid Aufwandmenge | Antidot (Tabelle 2) No | Aufwandmenge | Schutzwirkung |
|---|---|---|---|
| 100 g/hg | 140 | 400 g/ha | 38 % |
| | | 200 g/ha | 50 % |
| | | 100 g/ha | 50 % |
| | | 50 g/ha | 38 % |
| 400 g/hg | 143 | 400 g/ha | 63 % |
| | | 200 g/ha | 63 % |
| | | 100 g/ha | 50 % |
| | | 50 g/ha | 38 % |
| 200 g/hg | 143 | 400 g/ha | 75 % |
| | | 200 g/ha | 63 % |
| | | 100 g/ha | 63 % |
| | | 50 g/ha | 38 % |
| 100 g/hg | 143 | 400 g/ha | 38 % |
| | | 200 g/ha | 50 % |
| | | 100 g/ha | 50 % |
| | | 50 g/ha | 50 % |
| 400 g/hg | 144 | 400 g/ha | 38 % |
| | | 200 g/ha | 25 % |
| | | 100 g/ha | 38 % |
| | | 50 g/ha | 38 % |
| 200 g/hg | 144 | 400 g/ha | 63 % |
| | | 200 g/ha | 63 % |
| | | 100 g/ha | 63 % |
| | | 50 g/ha | 63 % |
| 100 g/hg | 144 | 400 g/ha | 38 % |
| | | 200 g/ha | 50 % |
| | | 100 g/ha | 50 % |
| | | 50 g/ha | 50 % |
| 400 g/hg | 149 | 400 g/ha | 50 % |
| | | 200 g/ha | 38 % |
| | | 100 g/ha | 38 % |
| | | 50 g/ha | 25 % |
| 200 g/hg | 149 | 400 g/ha | 63 % |
| | | 200 g/ha | 63 % |
| | | 100 g/ha | 63 % |
| | | 50 g/ha | 38 % |
| 100 g/hg | 149 | 400 g/ha | 50 % |
| | | 200 g/ha | 38 % |
| | | 100 g/ha | 38 % |
| | | 50 g/ha | 25 % |

| Herbizid Aufwandmenge | Antidot (Tabelle 2) No | Aufwandmenge | Schutzwirkung |
|---|---|---|---|
| 400 g/hg | 152 | 400 g/ha | 75 % |
| | | 200 g/ha | 75 % |
| | | 100 g/ha | 75 % |
| | | 50 g/ha | 50 % |
| 200 g/hg | 152 | 400 g/ha | 75 % |
| | | 200 g/ha | 75 % |
| | | 100 g/ha | 75 % |
| | | 50 g/ha | 63 % |
| 100 g/hg | 152 | 400 g/ha | 63 % |
| | | 200 g/ha | 63 % |
| | | 100 g/ha | 63 % |
| | | 50 g/ha | 50 % |
| 400 g/hg | 160 | 400 g/ha | 63 % |
| | | 200 g/ha | 75 % |
| | | 100 g/ha | 75 % |
| | | 50 g/ha | 63 % |
| 200 g/hg | 160 | 400 g/ha | 75 % |
| | | 200 g/ha | 75 % |
| | | 100 g/ha | 75 % |
| | | 50 g/ha | 63 % |
| 100 g/hg | 160 | 400 g/ha | 50 % |
| | | 200 g/ha | 50 % |
| | | 100 g/ha | 50 % |
| | | 50 g/ha | 50 % |

## Patentansprüche

1. Verwendung von 1,5-Diphenylpyrazol-3-carbonsäurederivaten zum Schützen von Kulturpflanzen vor der phytotoxischen Wirkung von herbizid wirksamen 2-[4-(Phenyl-, Pyridin-2-yl-, Benzoxazol-, Benzthiazol- und Chinoxalin-2-yl-oxy)-phenoxy]propionsäureestern, dadurch gekennzeichnet, dass man die Kulturpflanzen, Pflanzenteile oder die Kulturfläche mit einem 1,5-Diphenylpyrazol-3-carbonsäurederivat der Formel I behandelt

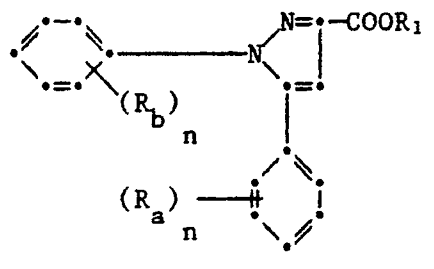

I

worin $R_a$ und $R_b$ unabhängig voneinander je Halogen, $C_1$-$C_5$-Alkyl, $C_1$-$C_5$-Halogenalkyl, $C_2$-$C_5$-Alkenyl, $C_2$-$C_5$-Alkinyl oder Cyano, n Null oder eine Zahl von 1 bis 3 und die Gruppe -$OR_1$ Hydroxy, einen pflanzenphysiologisch verträglichen Salz- oder einen beliebigen Esterrest bedeutet, worin
$R_1$ Wasserstoff, ein pflanzenphysiologisch verträgliches Metall- oder Ammoniumkation, $C_1$-$C_{18}$-Alkyl

oder $C_3$-$C_{18}$-Cycloalkyl unsubstituiert oder ein- oder mehrfach substituiert durch $C_2$-$C_8$-Alkenyl, $C_2$-$C_8$-Alkinyl, $C_3$-$C_8$-Cycloalkenyl, Halogen, Nitro, Cyan einen Rest $-XR_{10}$, $-CXR_{10}$, $-CX-XR_{10}$, $-CXNR_3R_4$, $-XCXR_{10}$, $-XCXNR_3R_4$, $-NR_3R_4$, $-NR_3CONR_3R_4$,$-CONR_3-NR_3R_4$, $-CONR_3-CONR_3R_4$, Si($C_1$-$C_4$alkyl)$_3$, $-C(OR_7)(OR_8)OR_9$, $PO(R_5)R_6$, einen Heterocyclus, Q der über C oder N gebunden ist, einen unsubstituierten oder substituierten Phenyl- oder Naphthylrest U, einen Rest-E-U oder einen Imidorest $-N=C(R_2)-R_2$,

$R_2$ $C_1$-$C_4$-Alkyl, $C_3$-$C_7$-Cycloalkyl oder die beiden $R_2$ zusammen einen 4-6 gliedrigen $C_4$-$C_{12}$-Alkylenrest der verzweigt und/oder durch Sauerstoff oder Schwefel unterbrochen sein kann

$R_3$ und $R_4$ unabhängig voneinander je Wasserstoff, $C_1$-$C_8$-Alkyl oder $C_3$-$C_8$-Cycloalkyl, unsubstituiert oder substituiert durch $C_2$-$C_6$-Alkenyl, $C_2$-$C_6$-Alkinyl, $C_3$-$C_7$-Cycloalkyl, Halogen, $C_1$-$C_8$-Alkoxy, $C_2$-$C_8$-Alkoxyalkoxy, $C_1$-$C_8$-Alkylthio, Cyan, eines von $C_3$ und $C_4$ auch $C_1$-$C_8$-Alkoxy, $-COOR_{10}$, $-CONH_2$, CONH($C_1$-$C_4$-Alkyl), CON($R_2$)$R_2$, $-NH_2$, -NH($C_1$-$C_2$-alkyl)-N($R_2$)$R_2$ einen Heterocyclus Q einen Phenyl- oder Napthylrest U, der direkt oder über eine $C_1$-$C_{14}$-Alkylenbrücke an das Stickstoffatom gebunden ist,

$R_3$ und $R_4$ zusammen eine 4-6-gliedrige $C_4$-$C_{12}$-Alkylen- oder Alkenylenkette, die verzweigt und/oder durch Sauerstoff oder Schwefel N($C_1$-$C_4$-Alkyl), N(Benzyl), $-SO_2-$ oder -CO- oder $-C(OR_7)OR_8$ unterbrochen sein kann und die durch Halogen, $C_1$-$C_4$-Alkyl, $C_1$-$C_4$-Alkoxy, $-NH_2$, -NH($C_1$-$C_4$-Alkyl), -N($C_1$-$C_4$-Alkyl)$_2$ substituiert sein kann,

$R_5$ und $R_6$ unabhängig voneinander je $C_1$-$C_4$-Alkyl, Hydroxy oder $C_1$-$C_4$-Alkoxy

$R_7$ und $R_8$ unabhängig voneinander je $C_1$-$C_4$-Alkyl,

$R_7$ und $R_8$ zusammen $C_2$-$C_4$-Alkylen

$R_9$ und $R_{10}$ je Wasserstoff, $C_1$-$C_8$-Alkyl oder $C_3$-$C_8$-Cycloalkyl unsubstituiert oder substituiert durch $C_2$-$C_6$-Alkenyl, $C_2$-$C_6$-Alkinyl oder $C_3$-$C_6$-Cycloalkenyl, Halogen, $C_1$-$C_4$-Alkoxy, $C_2$-$C_8$-Alkoxyalkoxy, $C_1$-$C_6$-Alkoxycarbonyl, $C_1$-$C_6$-Haloalkoxycarbonyl, einen Heterocyclus Q oder einen Phenyl- oder Naphthylrest U.

$R_9$ und $R_{10}$ bedeuten ferner den Phenyl- oder Naphthylrest U oder einen über C-gebundenen Heterocyclus Q,

Q ein gesättigten oder ungesättigten 5-12-gliedrigen Heterocyclus, der 1-4 Heteroatome N, O, S resp. eine -SO-, $-SO_2-$, N($C_1$-$C_4$-Allyl)-oder -N(Benzyl)-Gruppe enthält wobei Sauerstoff und Schwefel nicht in vicinaler Stellung befinden dürfen, und der durch Halogen, $C_1$-$C_4$-Alkyl, $C_1$-$C_4$-Alkoxy, $C_1$-$C_4$-Haloalkyl, $C_1$-$C_4$-Haloalkoxy, Cyan, Nitro substituiert sein kann,

E eine $C_1$-$C_4$ Alkylen-, $C_2$-$C_4$ Alkenylen- oder $C_2$-$C_4$ Alkinylenbrücke,

U ein Phenyl- oder Naphthylrest, der unsubstituiert oder ein oder mehrfach substituiert ist durch Halogen, $C_1$-$C_4$-Alkyl, X $C_1$-$C_4$-Alkyl, $C_1$-$C_4$-Alkylsulfinyl, $C_1$-$C_4$-Alkylsulfonyl, $C_1$-$C_4$-Haloalkyl, $C_1$-$C_4$-Haloalkoxy, Cyano, Nitro, -COOH, $-COOC_1$-$C_4$-Alkyl, $-COC_1$-$C_4$-Alkyl, $CONH_2$, CONH($C_1$-$C_4$-Alkyl), CON($C_1$-$C_4$-Alkyl)$_2$, $-SO_2NH_2$, $SO_2NH$($C_1$-$C_4$-Alkyl), $SO_2N$($C_1$-$C_4$-Alkyl), Pyrrolidino, Piperidino oder durch den Pyrrolidinocarbonyl, Piperidinocarbonyl, Morpholinocarbonylrest,

X Sauerstoff oder Schwefel

bedeuten und die Kultur oder Kulturfläche gleichzeitig oder nachfolgend mit einem 2-[4-(Phenyloxy, Pyridin-2-yloxy, Benzoxazolyloxy, Benzthiazolyloxy oder Chinoxalin-2-yloxy)phenoxy]propionsäureester der Formel II behandelt

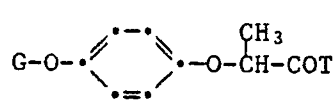 (II),

worin G

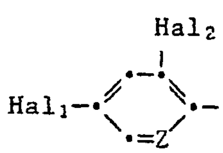 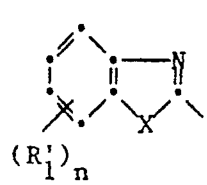 oder 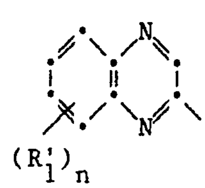 ,

$Hal_1$ Fluor, Chlor, Brom, Jod oder Trifluoromethyl, $Hal_2$ Wasserstoff, Fluor, Chlor, Brom oder Trifluoromethyl, Z für Stickstoff oder Methin(-CH=), X ein Sauerstoff oder Schwefelatom, $R_1'$ Halogen, Trifluormethyl, Nitro, Cyan, $C_1$-$C_4$-Alkyl oder $C_1$-$C_4$-Alkoxy und n 0, 1, 2 oder 3 bedeuten, und
T für

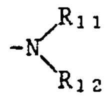 , 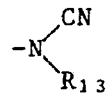 ,

-$OR_{14}$ oder -O-N=$CR_{15}R_{16}$ steht, worin
$R_{11}$, $R_{12}$ unabhängig voneinander Wasserstoff, $C_1$-$C_8$-Alkoxy, $C_1$-$C_8$-Alkyl, Phenyl oder Benzyl, $R_{11}$ und $R_{12}$ zusammen mit dem sie tragenden Stickstoffatom einen 5-bis 6-gliedrigen gesättigten Stickstoffheterocyclus, der durch ein Sauerstoff- oder Schwefelatom unterbrochen sein kann,
$R_{13}$ $C_1$-$C_4$-Alkyl, $C_3$-$C_4$-Alkenyl, $C_3$-$C_4$-Alkinyl oder $C_2$-$C_4$-Alkoxyalkyl,
$R_{14}$ Wasserstoff oder das Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, -COOR, -COSR, - $CONH_2$-, CON($C_1$-$C_4$-alkoxy)-$C_1$-$C_4$-alkyl, -CO-N-di-$C_1$-$C_4$-alkyl, -CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest; $C_3$-$C_9$-Cycloalkyl; oder gegebenenfalls durch Cyan, $C_1$-$C_4$-Alkyl, $C_1$-$C_4$-Alkoxy, Acetyl, -$COOR_{17}$, -$COSR_{17}$, -$CONH_2$, -CON($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,
und $R_{16}$ unabhängig voneinander $C_1$-$C_4$-Alkyl oder zusammen eine 3-bis 6-gliedrige Alkylenkette und
$R_{17}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkenyl, $C_3$-$C_6$-Alkinyl oder $C_3$-$C_6$-Halogenalkinyl bedeuten.

2. Verfahren zur Bekämpfung von Unkräutern in Nutzpflanzenkulturen, dadurch gekennzeichnet, dass man die Kulturen oder deren Anbaufläche geichzeitig oder in kurzer Zeitfolge mit einer herbizid wirksamen Menge eines 2-[4-Phenyloxy, Pyridin-2-yloxy, Benzoxazolyloxy, Benzthiazolyloxy oder Chinoxalin-2-yl-oxy)phenoxy]propionsäureesters der Formel II gemäss Anpruch 1 und als Gegenmittel einer wirksamen Menge eines 1,5-Diphenylpyrazol-3-carbonsäurederivates der Formel I gemäss Anspruch 1 behandelt.

3. Selektives herbizides Mittel zur Bekämpfung von Unkräutern in Nutzpflanzenkulturen, dadurch gekennzeichnet, dass es als herbizid wirksame Komponente einen 2-[4-(Phenyloxy, Pyridin-2-yloxy, Benzoxazolyloxy, Benzthiazolyloxy oder Chinoxalin-2-yl-oxy)phenoxy]-propionsäureester der Formel II gemäss Anspruch 1 und als Gegenmittel eine wirksame Menge eines 1,5-Diphenylpyrazol-3-carbonsäurederivates der Formel I gemäss Anspruch 1 zusammen mit inerten Trägermaterialien und Hilfsstoffen enhält.

4. Mittel gemäss Anspruch 3, dadurch gekennzeichnet, dass es als herbizid wirksame Komponente einen 4-(4-Chlor-2-fluorpyridin-2-yloxy)phenoxy]-propionsäureester der Formel II enthält,

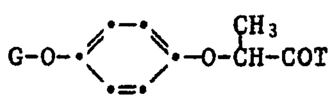 (II),

worin G den 4-Chlor-2-fluorpyridin-2-ylrest

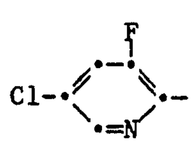

und T einen Rest ausgewählt aus Methoxy, Aethoxy, Propyloxy, Isopropyloxy, Butyloxy, Dimethylaminoäthyloxy, Propargyloxy, 1-Cyano-1-methyläthoxy, Methylcarbonylmethylthio, 1-Aethoxycarbonyläthoxy, Butoxycarbonyl, Acetoximoxy, Methyläthylketoximoxy, Cyclohexanoximoxy bedeutet, und als Gegenmittel ein 1,5-Diphenylpyrazol-3-carbonsäurederivat der Formel I gemäss Anspruch 1, zusammen mit inerten Trägermaterialien und Hilfsstoffen enthält.

5. Verfahren zur selektiven Bekämpfung von Unkräutern in Nutzpflanzenkulturen, dadurch gekennzeichnet, dass man die Kulturen oder deren Anbaufläche mit einem Mittel gemäss Anspruch 4 behandelt.

6. Mittel gemäss Anspruch 3, dadurch gekennzeichnet, dass es als herbizid wirksame Komponente einen 2-[4-(7-Chlorbenzoxazol-2-yloxy)phenoxy]propionsäureester der Formel II

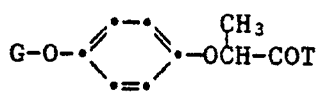

(II)

worin G den 7-Chlorbenzoxazol-2-ylrest

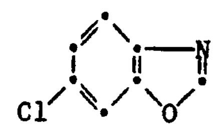

und T einen Rest ausgewählt aus Methoxy, Aethoxy, Propyloxy, Isopropyloxy, Butyloxy, Dimethylaminoäthyloxy, Propargyloxy, 1-Cyano-1-methyloxy, Methoxycarbonylmethylthio, 1-Aethoxycarbonyläthoxy, Butoxycarbonyl, Acetoxim-oxy, Methyläthylketoxim-oxy und Cyclohexanoxim-oxy bedeutet, und als Gegenmittel ein 1,5-Diphenylpyrazolcarbonsäurederivat der Formel I, Anspruch 1, zusammen mit inerten Trägermaterialien und Hilfsstoffen enthält.

7. Verfahren zur selektiven Bekämpfung von Unkräutern in Nutzpflanzenkulturen, dadurch gekennzeichnet, dass man die Kulturen oder deren Anbaufläche mit einem Mittel gemäss Anspruch 6 behandelt.

8. Mittel gemäss Anspruch 3, dadurch gekennzeichnet, dass es als herbizid wirksame Komponente einen 2-[4-(7-Chlorchinoxalin-2-yloxy)-phenoxy]-propionsäureester der Formel II

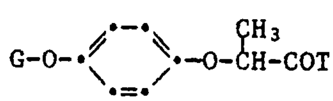

(II)

worin G den 7-Chlorchinoxalin-2-ylrest

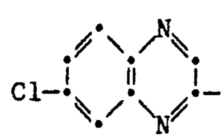

und T einen Rest ausgewählt aus Methoxy, Aethoxy, Propyloxy, Isopropyloxy, Butyloxy, Dimethylaminoäthoxy, Propargyloxy, 1-Cyano-1-methyläthoxy, Methoxycarbonylmethylthio, 1-Aethylcarbonyläthoxy, Butyloxycarbonyl, Acetoxim-oxy, Methyläthylketoxim-oxy und Cyclohexanonoxim-oxy bedeutet, und als Gegenmittel ein 1,5-Diphenylpyrazolcarbonsäurederivat der Formel I gemäss Anspruch 1, zusammen mit inerten Trägermaterialien und Hilfsstoffen enthält.

9. Verfahren zur selektiven Bekämpfung von Unkräutern in Nutzpflanzenkulturen, dadurch gekennzeichnet, dass man die Kulturen oder deren Anbaufläche mit einem Mittel gemäss Anspruch 8 behandelt.

10. Mittel gemäss Anspruch 3, dadurch gekennzeichnet, dass es als herbizid wirksame Komponente 2-[4-(7-Chlorbenzoxazol-2-yloxy)-phenoxy]-propionsäureäthylester der Formel

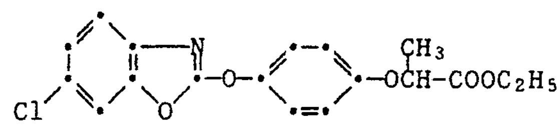

enthält und als Gegenmittel ein 1,5-Diphenylpyrazol-3-carbonsäurederivat der Formel I gemäss Anspruch 1 zusammen mit inerten Trägermaterialien und Hilfsstoffen.

**11.** Verfahren zur selektiven Bekämpfung von Unkräutern in Nutzpflanzenkulturen, dadurch gekennzeichnet, dass man die Kulturen oder deren Anbaufläche mit einem Mittel gemäss Anspruch 10 behandelt.

**12.** Mittel gemäss Anspruch 3, dadurch gekennzeichnet, dass es als herbizid wirksame Komponente 2-[4-(7-Chlor-chinoxalin-2-yloxy)-phenoxy]-propionsäureäthylester der Formel

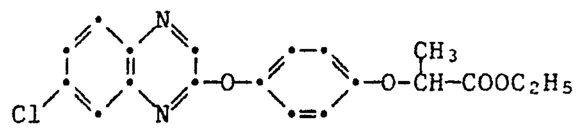

enthält und als Gegenmittel ein 1,5-Diphenylpyrazol-3-carbonsäurederivat der Formel I gemäss Anspruch 1, zusammen mit inerten Trägermaterialien und Hilfsstoffen.

**13.** Verfahren zum selektiven Bekämpfen von Unkräutern in Nutzpflanzenkulturen, dadurch gekennzeichnet, dass man die Kulturen oder deren Anbaufläche mit einem Mittel gemäss Anspruch 12 behandelt.

**14.** Mittel gemäss Anspruch 3, dadurch gekennzeichnet, dass es als herbizid wirksame Komponente 2-[4-(5-Chlor-3-fluorpyridin-2-yloxy)-phenoxy]-propionsäure-propargylester der Formel

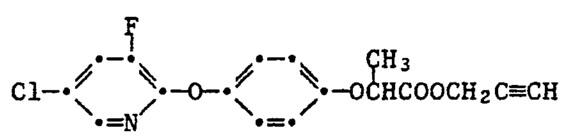

oder sein 2R-Enantiomer enthält und als Gegenmittel ein 1,5-Diphenylpyrazol-3-carbonsäurederivat der Formel I gemäss Anspruch 1, zusammen mit inerten Trägermaterialien und Hilfsstoffen.

**15.** Verfahren zur selektiven Bekämpfung von Unkräutern in Nutzpflanzenkulturen, dadurch gekennzeichnet, dass man die Kulturen oder deren Anbaufläche mit einem Mittel gemäss Anspruch 14 behandelt.

**16.** Neue 1,5-Diphenylpyrazol-3-carbonsäurederivate der Formel (I)

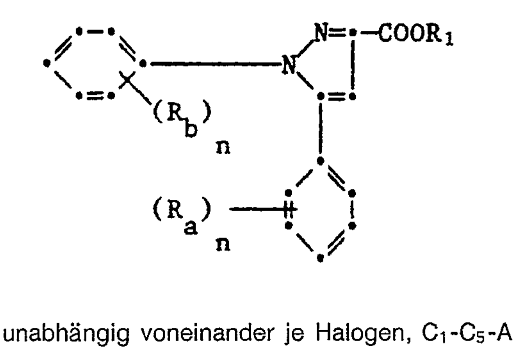

I

worin $R_a$ und $R_b$ unabhängig voneinander je Halogen, $C_1$-$C_5$-Alkyl, $C_1$-$C_5$-Halogenalkyl, $C_2$-$C_5$-Alkenyl, $C_2$-$C_5$-Alkinyl oder Cyano, n Null oder eine Zahl von 1 bis 3 und die Gruppe -$OR_1$ Hydroxy oder einen pflanzenphysiologisch verträglichen Salz- oder einen beliebigen Esterrest bedeutet, worin $R_1$ Wasserstoff, ein pflanzenphysiologisch verträgliches Metall- oder Ammoniumkation,

$C_1$-$C_{18}$-Alkyl oder $C_3$-$C_{18}$-Cycloalkyl unsubstituiert oder ein- oder mehrfach substituiert durch $C_2$-$C_8$-Alkenyl, $C_2$-$C_8$-Alkinyl, $C_3$-$C_8$-Cycloalkenyl, Halogen, Nitro, Cyan, einen Rest -$XR_{10}$, -$CXR_{10}$, -CX-$XR_{10}$, -$CXNR_3R_4$, -$XCXR_{10}$, -$XCXNR_3R_4$, -$NR_3R_4$, -$NR_3CONR_3R_4$, -$CONR_3$-$NR_3R_4$, -$CONR_3$-$CONR_3COR_4$, Si($C_1$-$C_4$alkyl)$_3$, -C($OR_7$)($OR_8$)$OR_9$, PO($R_5$)$R_6$, einen Heterocyclus, Q der über C oder N gebunden ist, einen unsubstituierten oder substituierten Phenyl- oder Naphthylrest U, einen Rest-E-U oder einem Imidorest -N = C($R_2$)$R_2$,

$R_2$ $C_1$-$C_4$-Alkyl, $C_3$-$C_7$-Cycloalkyl oder die beiden $R_2$ zusammen einen 4-6 gliedrigen $C_4$-$C_{12}$-Alkylenrest der verzweigt und/oder durch Sauerstoff oder Schwefel unterbrochen sein kann

$R_3$ und $R_4$ unabhängig voneinander je Wasserstoff, $C_1$-$C_8$-Alkyl oder $C_3$-$C_8$-Cycloalkyl, unsubstituiert oder substituiert durch $C_2$-$C_6$-Alkenyl, $C_2$-$C_6$-Alkinyl, $C_3$-$C_7$-Cycloalkyl, Halogen, $C_1$-$C_8$-Alkoxy, $C_2$-$C_8$-Alkoxyalkoxy, $C_1$-$C_8$-Alkylthio, Cyan, eines von $C_3$ und $C_4$ auch $C_1$-$C_8$-Alkoxy, -$COOR_{10}$, -$CONH_2$, CONH($C_1$-$C_4$-Alkyl), CON($R_2$)$R_2$, -$NH_2$, -NH($C_1$-$C_2$-alkyl)-N($R_2$)$R_2$ einen Heterocyclus Q einen Phenyl- oder Napthylrest U, der direkt oder über eine $C_1$-$C_{14}$-Alkylenbrücke an das Stickstoffatom gebunden ist,

$R_3$ und $R_4$ zusammen eine 4-6-gliedrige $C_4$-$C_{12}$-Alkylen- oder Alkenylenkette, die verzweigt und/oder durch Sauerstoff oder Schwefel N($C_1$-$C_4$-Alkyl), N(Benzyl), -$SO_2$- oder -CO- oder -C($OR_7$)$OR_8$ unterbrochen sein kann und die durch Halogen, $C_1$-$C_4$-Alkyl, $C_1$-$C_4$-Alkoxy, -$NH_2$, -NH($C_1$-$C_4$-Alkyl), -N($C_1$-$C_4$-Alkyl)$_2$ substituiert sein kann,

$R_5$ und $R_6$ unabhängig voneinander je $C_1$-$C_4$-Alkyl, Hydroxy oder $C_1$-$C_4$-Alkoxy

$R_7$ und $R_8$ unabhängig voneinander je $C_1$-$C_4$-Alkyl,

$R_7$ und $R_8$ zusammen $C_2$-$C_4$-Alkylen

$R_9$ und $R_{10}$ je Wasserstoff, $C_1$-$C_8$-Alkyl oder $C_3$-$C_8$-Cycloalkyl unsubstituiert oder substituiert durch $C_2$-$C_6$-Alkenyl, $C_2$-$C_6$-Alkinyl oder $C_3$-$C_6$-Cycloalkenyl, Halogen, $C_1$-$C_4$-Alkoxy, $C_2$-$C_8$-Alkoxyalkoxy, $C_1$-$C_6$-Alkoxycarbonyl, $C_1$-$C_6$-Haloalkoxycarbonyl, einen Heterocyclus Q oder einen Phenyl- oder Naphthylrest U.

$R_9$ und $R_{10}$ bedeuten ferner den Phenyl- oder Naphthylrest U oder einen über C-gebundenen Heterocyclus Q,

Q ein gesättigten oder ungesättigten 5-12-gliedrigen Heterocyclus, der 1-4 Heteroatome N, O, S resp. eine -SO-, -$SO_2$-, N($C_1$-$C_4$-Allyl)-oder -N(Benzyl)-Gruppe enthält wobei Sauerstoff und Schwefel nicht in vicinaler Stellung befinden dürfen, und der durch Halogen, $C_1$-$C_4$-Alkyl, $C_1$-$C_4$-Alkoxy, $C_1$-$C_4$-Haloalkyl, $C_1$-$C_4$-Haloalkoxy, Cyan, Nitro substituiert sein kann,

E eine $C_1$-$C_4$ Alkylen-, $C_2$-$C_4$ Alkenylen- oder $C_2$-$C_4$ Alkinylenbrücke,

U ein Phenyl- oder Naphthylrest, der unsubstituiert oder ein oder mehrfach substituiert ist durch Halogen, $C_1$-$C_4$-Alkyl, X $C_1$-$C_4$-Alkyl, $C_1$-$C_4$-Alkylsulfinyl, $C_1$-$C_4$-Alkylsulfonyl, $C_1$-$C_4$-Haloalkyl, $C_1$-$C_4$-Haloalkoxy, Cyano, Nitro, -COOH, -$COOC_1$-$C_4$-Alkyl, -$COC_1$-$C_4$-Alkyl, $CONH_2$, CONH($C_1$-$C_4$-Alkyl), CON($C_1$-$C_4$-Alkyl)$_2$, -$SO_2NH_2$, $SO_2$NH($C_1$-$C_4$-Alkyl), $SO_2$N($C_1$-$C_4$-Alkyl), Pyrrolidino, Piperidino oder durch den Pyrrolidinocarbonyl, Piperidinocarbonyl, Morpholinocarbonylrest,

X Sauerstoff oder Schwefel

bedeuten mit der Massgabe, dass wenn ($R_a$) Wasserstoff, n = 1 und $R_1$ Wasserstoff oder Aethyl ist, ($R_b$) falls es Methyl oder Halogen bedeutet, in der ortho-Stellung sein muss.

**17.** 1,5-Diphenylpyrazol-3-carbonsäurederivate der Formel I, Anspruch 16 dadurch gekennzeichnet, dass $R_a$, $R_b$ und n die in Anspruch 1 gegebene Bedeutung haben, während $R_1$ Wasserstoff, ein pflanzenphysiologisch verträgliches Metall- oder Ammoniumkation, $C_1$-$C_{18}$-Alkyl oder $C_3$-$C_{18}$-Cycloalkyl unsubstituiert oder ein- oder mehrfach substituiert durch $C_2$-$C_8$-Alkenyl, $C_2$-$C_8$-Alkinyl, $C_3$-$C_8$-Cycloalkenyl, Halogen, Nitro, Cyan, $C_1$-$C_4$-Alkoxy, $C_1$-$C_4$-Alkoxycarbonyl, $C_1$-$C_4$-Alkylcarbonyl, Carbamoyl, Di($C_1$-$C_4$-Alkylcarbamoyl), Amino, $C_1$-$C_4$-Alkylamino, Di($C_1$-$C_4$-Alkylamino) einen 5-6-gliedrigen Heterocyclus Q der über N oder C gebunden ist oder einen Phenyl-oder Napthylrest U einen Rest-E-U oder einen Imidorest -N = C($R_2$)$R_2$ wobei E, $R_2$ Q und U die im Anspruch 16 gegebene Bedeutung haben mit der Massgabe, dass wenn ($R_a$) Wasserstoff, n = 1 und $R_1$ Wasserstoff oder Aethyl sind, ($R_b$) falls es Methyl oder Halogen bedeutet, in der ortho-Stellung sein muss.

**18.** 1,5-Diphenylpyrazol-3-carbonsäureester der Formel I, Anspruch 16, worin $R_a$, $R_b$ und n die im Anspruch 1 gegebene Bedeutung haben während $R_1$ $C_1$-$C_{18}$-Alkyl unsubstituiert oder ein- oder mehrfach substituiert durch $C_2$-$C_8$-Alkyl, $C_2$-$C_8$-Alkinyl, Halogen, Nitro, Cyan, $C_1$-$C_4$-Alkoxy oder Phenyl bedeutet.

**19.** 1,5-Diphenylpyrazol-3-carbonsäuresalze der Formel I Anspruch 16, worin $R_a$, $R_b$ und n die im Anspruch 1 gegebene Bedeutung haben und $R_1$ ein pflanzenphysiologisch verträgliches Metall- oder Ammoniumkation bedeutet.

**20.** 1-(2-Chlorphenyl)-3-methoxycarbonyl-5-phenylpyrazol gemäss Anspruch 16.

**21.** 1-(2,4-Dichlorphenyl)-3-methoxycarbonyl-5-phenylpyrazol gemäss Anspruch 16.

**22.** 1-(2-Chlorphenyl)-3-methoxycarbonyl-5-(2-fluorphenyl)-pyrazol gemäss Anspruch 16.

**23.** Verfahren zur Herstellung der 1,5-Diphenylpyrazol-3-carbonsäuren und Derivaten der Formel I gemäss Anspruch 16, dadurch gekennzeichnet, dass man ein Acetophenon der Formel III

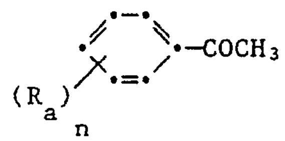

(III)

worin $R_a$ und n die unter der Formel I gegebene Bedeutung haben, in einem inerten organischen Lösungsmittel in Gegenwart einr Base mit der äquimolaren Menge eines Oxalsäurediesters der Formel IV umsetzt

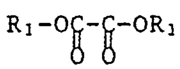

(IV)

worin $R_1$ die unter der Formel I gegebene Bedeutung hat, den entstandene Benzoylbenztraubensäureester der Formel V

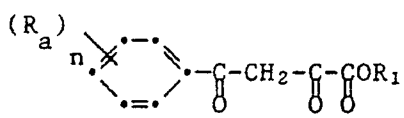

(V)

worin $R_a$, $R_1$ und n die unter der Formel I gegebene Bedeutung haben mit der äquimolaren Menge Phenylhydrazin der Formel VI versetzt

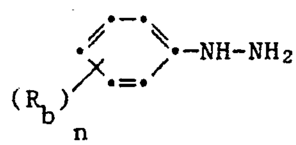

(VI)

worin $R_b$ und n die unter der Formel I gegebene Bedeutung haben, worauf man das Gemisch in saurem Milieu bei erhöhter Temperatur zum 1,5-Diphenylpyrazolcarbonsäurederivat der Formel I cyclisiert.

**24.** Verfahren zur Herstellung der 1,5-Diphenylpyrazol-3-carbonsäurederivate der Formel I gemäss Anspruch 16, dadurch gekennzeichnet, dass man in einem inerten Lösungs- oder Verdünnungsmittel ein 1,5-Diphenylpyrazol-3-carbonsäurehalogenid der Formel VII

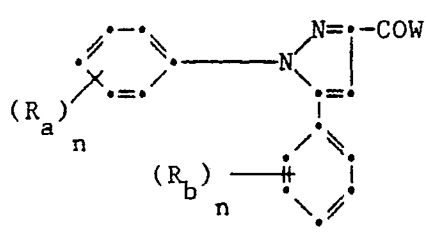

(VII),

worin n, $R_a$ und $R_b$ die in Anspruch 16 gegebene Bedeutung haben und W für ein Halogenatom Chlor oder Brom steht, mit einem Alkohol der Formel VIII oder einem Metall- oder Ammoniumsalz davon umsetzt,

$R_1OH$ (VIII),

worin $R_1$ die im Anspruch 16 gegebene Bedeutung hat.

**25.** Neue 1,5-Diphenylpyrazol-3-carbonsäurehalogenide der Formel VII

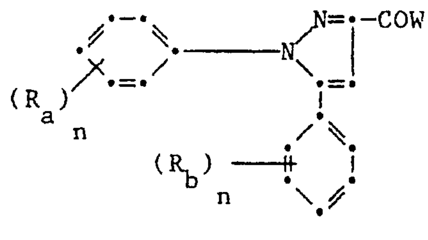

(VII),

worin n, $R_a$ und $R_b$ die im Anspruch 16 gegebene Bedeutung haben und W für ein Halogenatom Chlor oder Brom steht, als Zwischenprodukte für die Herstellung der 1,5-Diphenylpyrazol-3-carbonsäurederivate gemäss Anspruch 16.

## Claims

**1.** The use of 1,5-diphenylpyrazole-3-carboxylic acid derivatives for the protection of cultivated plants from the phytotoxic action of herbicidally active 2-[4-(phenoxy, pyridin-2-yloxy, benzoxazolyloxy, benzothiazolyloxy and quinoxalin-2-yloxy)-phenoxy]-propionic acid esters, characterised in that the cultivated plants, plant parts or the crop area are(is) treated with a 1,5-diphenylpyrazole-3-carboxylic acid derivative of the formula I

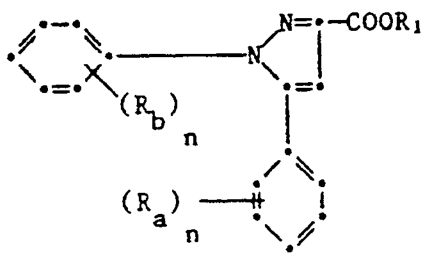

I

in which each of $R_a$ and $R_b$, independently of the other, represents halogen, $C_1$-$C_5$-alkyl, $C_1$-$C_5$-haloalkyl, $C_2$-$C_5$-alkenyl, $C_2$-$C_5$-alkynyl or cyano, n represents zero or a number from 1 to 3 and the group $-OR_1$ represents hydroxy, a plant-physiologically tolerable salt radical or any desired ester radical, in which

$R_1$ represents hydrogen, a plant-physiologically tolerable metal or ammonium cation, or $C_1$-$C_{18}$-alkyl or $C_3$-$C_{18}$-cycloalkyl each of which is unsubstituted or mono- or poly-substituted by $C_2$-$C_8$-alkenyl, $C_2$-$C_8$-alkynyl, $C_3$-$C_8$-cycloalkenyl, halogen, nitro, cyano or by an $-XR_{10}$, $-CXR_{10}$, $-CX-XR_{10}$, $-CXNR_3R_4$, $-XCXR_{10}$, $-XCXNR_3R_4$, $-NR_3R_4$, $-NR_3CONR_3R_4$, $-CONR_3-NR_3R_4$, $-CONR_3-CONR_3R_4$, $-Si(C_1-C_4\text{-alkyl})_3$, $-C(OR_7)(OR_8)OR_9$ or $PO(R_5)R_6$ radical; a heterocycle Q that is bonded via C or N; an unsubstituted or substituted phenyl or naphthyl radical U, a radical E-U or an imido radical $-N=C(R_2)R_2$,

$R_2$ represents $C_1$-$C_4$-alkyl or $C_3$-$C_7$-cycloalkyl, or the two radicals $R_2$ together represent a 4- to 6-membered $C_4$-$C_{12}$-alkylene radical that may be branched and/or interrupted by oxygen or sulphur,

each of $R_3$ and $R_4$, independently of the other, represents hydrogen, or $C_1$-$C_8$-alkyl or $C_3$-$C_8$-cycloalkyl each of which is unsubstituted or substituted by $C_2$-$C_6$-alkenyl, $C_2$-$C_6$-alkynyl, $C_3$-$C_7$-cycloalkyl, halogen, $C_1$-$C_8$-alkoxy, $C_2$-$C_8$-alkoxyalkoxy, $C_1$-$C_8$-alkylthio or by cyano, and one of $C_3$ and $C_4$ alternatively represents $C_1$-$C_8$-alkoxy, $-COOR_{10}$, $-CONH_2$, $CONH(C_1-C_4\text{-alkyl})$, $CON(R_2)R_2$, $-NH_2$, $-NH-(C_1-C_2\text{-alkyl})-N(R_2)R_2$, a heterocycle Q, or a phenyl or naphthyl radical U that is bonded to the nitrogen

atom directly or via a $C_1$-$C_{14}$-alkylene bridge,
$R_3$ and $R_4$ together represent a 4- to 6-membered $C_4$-$C_{12}$-alkylene or -alkenylene chain that may be branched and/or interrupted by oxygen, sulphur, N($C_1$-$C_4$-alkyl), N(benzyl), -$SO_2$-, -CO- or by -C($OR_7$)-$OR_8$, and that may be substituted by halogen, $C_1$-$C_4$-alkyl, $C_1$-$C_4$-alkoxy, -$NH_2$, -NH($C_1$-$C_4$-alkyl) or by -N($C_1$-$C_4$-alkyl$)_2$,
each of $R_5$ and $R_6$, independently of the other, represents $C_1$-$C_4$-alkyl, hydroxy or $C_1$-$C_4$-alkoxy,
each of $R_7$ and $R_8$, independently of the other, represents $C_1$-$C_4$-alkyl,
$R_7$ and $R_8$ together represent $C_2$-$C_4$-alkylene,
each of $R_9$ and $R_{10}$ represents hydrogen, or $C_1$-$C_8$-alkyl or $C_3$-$C_8$-oycloalkyl each of which is unsubstituted or substituted by $C_2$-$C_6$-alkenyl, $C_2$-$C_6$-alkynyl, $C_3$-$C_6$-cycloalkenyl, halogen, $C_1$-$C_4$-alkoxy, $C_2$-$C_8$-alkoxyalkoxy, $C_1$-$C_6$-alkoxycarbonyl, $C_1$-$C_6$-haloalkoxycarbonyl, a heterocycle Q or by a phenyl or naphthyl radical U,
$R_9$ and $R_{10}$ also represent the phenyl or naphthyl radical U, or a heterocycle Q bonded via C,
Q represents a saturated or unsaturated 5- to 12-membered heterocycle that contains from 1 to 4 hetero atoms N, O, S or a -SO-, -$SO_2$-, N($C_1$-$C_4$-allyl) or N(benzyl) group, wherein oxygen and sulphur must not be in vicinal positions, and that may be substituted by halogen, $C_1$-$C_4$-alkyl, $C_1$-$C_4$-alkoxy, $C_1$-$C_4$-haloalkyl, $C_1$-$C_4$-haloalkoxy, cyano or by nitro,
E represents a $C_1$-$C_4$-alkylene bridge, a $C_2$-$C_4$-alkenylene bridge or a $C_2$-$C_4$-alkynylene bridge,
U represents a phenyl or naphthyl radical that is unsubstituted or mono- or poly-substituted by halogen, $C_1$-$C_4$-alkyl, X $C_1$-$C_4$-alkyl, $C_1$-$C_4$-alkylsulphinyl, $C_1$-$C_4$-alkylsulphonyl, $C_1$-$C_4$-haloalkyl, $C_1$-$C_4$-haloalkoxy, cyano, nitro, -COOH, -$COOC_1$-$C_4$-alkyl, -$COC_1$-$C_4$-alkyl, $CONH_2$, CONH($C_1$-$C_4$-alkyl), CON($C_1$-$C_4$-alkyl$)_2$, -$SO_2NH_2$, $SO_2NH$($C_1$-$C_4$-alkyl), $SO_2N$($C_1$-$C_4$-alkyl), pyrrolidino, piperidino or by the pyrrolidinocarbonyl, piperidinocarbonyl or morpholinocarbonyl radical,
and X represents oxygen or sulphur,
and the crop or crop area is treated at the same time or subsequently with a 2-[4-(phenoxy, pyridin-2-yloxy, benzoxazolyloxy, benzothiazolyloxy or quinoxalin-2-yloxy)-phenoxy]-propionic acid ester of the formula II

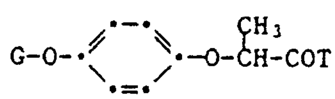 (II),

in which G represents

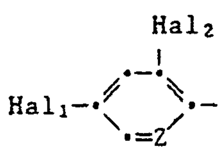 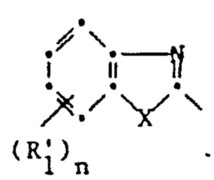 . or 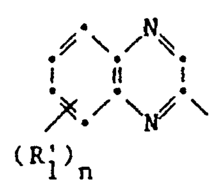 ,

in which $Hal_1$ represents fluorine, chlorine, bromine, iodine or trifluoromethyl, $Hal_2$ represents hydrogen, fluorine, chlorine, bromine or trifluoromethyl, Z represents nitrogen or methine (-CH=), X represents an oxygen or sulphur atom, $R_1'$ represents halogen, trifluoromethyl, nitro, cyano, $C_1$-$C_4$-alkyl or $C_1$-$C_4$-alkoxy and n represents 0, 1, 2 or 3, and
T represents

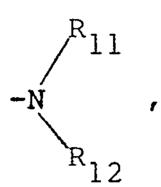 , 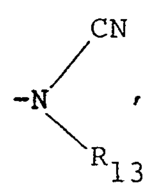 ,

-$OR_{14}$ or -O-N=$CR_{15}R_{16}$
in which

each of $R_{11}$ and $R_{12}$, independently of the other, represents hydrogen, $C_1$-$C_8$-alkoxy, $C_1$-$C_8$-alkyl, phenyl or benzyl, and $R_{11}$ and $R_{12}$ together with the nitrogen atom carrying them represent a 5- or 6-membered saturated nitrogen heterocycle that may be interrupted by an oxygen or sulphur atom,
$R_{13}$ represents $C_1$-$C_4$-alkyl, $C_3$-$C_4$-alkenyl, $C_3$-$C_4$-alkynyl or $C_2$-$C_4$-alkoxyalkyl,
$R_{14}$ represents hydrogen or the equivalent of an alkali metal ion, an alkaline earth metal ion, a copper ion or an iron ion; a quaternary $C_1$-$C_4$-alkylammonium or $C_1$-$C_4$-hydroxyalkylammonium radical; a $C_1$-$C_9$-alkyl radical that is unsubstituted or mono- or poly-substituted by amino, halogen, hydroxy, cyano, nitro, phenyl, $C_1$-$C_4$-alkoxy, polyethoxy having from 2 to 6 ethylene oxide units, -COOR, -COSR, -CONH$_2$-, -CON($C_1$-$C_4$-alkoxy)-$C_1$-$C_4$-alkyl, -CO-N-di-$C_1$-$C_4$-alkyl, -CONH-$C_1$-$C_4$-alkyl, -N($C_1$-$C_4$-alkoxy)-$C_1$-$C_4$-alkyl or by di-$C_1$-$C_4$-alkylamino; a $C_3$-$C_9$-alkenyl radical that is unsubstituted or substituted by halogen or by $C_1$-$C_4$-alkoxy; a $C_3$-$C_9$-alkynyl radical that is unsubstituted or substituted by halogen or by $C_1$-$C_4$-alkoxy; $C_3$-$C_9$-cycloalkyl; or phenyl that is unsubstituted or substituted by cyano, $C_1$-$C_4$-alkyl, $C_1$-$C_4$-alkoxy, acetyl, -COOR$_{17}$, -COSR$_{17}$, -CONH$_2$, -CON($C_1$-$C_4$-alkoxy) -$C_1$-$C_4$-alkyl, -CO-N-di-$C_1$-$C_4$-alkyl or by -CONH-$C_1$-$C_4$-alkyl,
and $R_{16}$, independently of the other, represents $C_1$-$C_4$-alkyl or they together represent a 3- to 6-membered alkylene chain and
$R_{17}$ represents hydrogen, $C_1$-$C_6$-alkyl, $C_1$-$C_6$-haloalkyl, $C_2$-$C_6$-alkoxyalkyl, $C_3$-$C_6$-alkenyl, $C_3$-$C_6$-haloalkenyl, $C_3$-$C_6$-alkynyl or $C_3$-$C_6$-haloalkynyl.

2. A method of controlling weeds in crops of useful plants, characterised in that the crops or the areas in which they are grown are treated with a herbicidally effective amount of a 2-[4-phenoxy, pyridin-2-yloxy, benzoxazolyloxy, benzothiazolyloxy or quinoxalin-2-yl-oxy)-phenoxy]-propionic acid ester of the formula II according to claim 1 and at the same time or shortly afterwards with an effective amount of a 1,5-diphenylpyrazole-3-carboxylic acid derivative of the formula I according to claim 1 as safener.

3. A selective herbicidal composition for controlling weeds in crops of useful plants, characterised in that it comprises as herbicidally active component a 2-[4-(phenoxy, pyridin-2-yloxy, benzoxazolyloxy, benzothiazolyloxy or quinoxalin-2-yloxy)-phenoxy]-propionic acid ester of formula II according to claim 1 and, as safener, an effective amount of a 1,5-diphenylpyrazole-3-carboxylic acid derivative of the formula I according to claim 1 together with inert carriers and adjuvants.

4. A composition according to claim 3, characterised in that it comprises as herbicidally active component a 4-(4-chloro-2-fluoropyridin-2-yloxy)-phenoxy]-propionic acid ester of the formula II

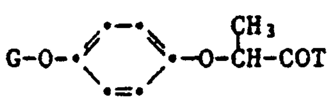

(II),

in which G represents a 4-chloro-2-fluoropyridin-2-yl radical

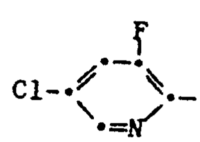

and T represents a radical selected from methoxy, ethoxy, propoxy, isopropoxy, butoxy, dimethylaminoethoxy, propargyloxy, 1-cyano-1-methylethoxy, methylcarbonylmethylthio, 1-ethoxycarbonylethoxy, butoxycarbonyl, acetoximoxy, methylethylketoximoxy and cyclohexanoximoxy and, as safener, a 1,5-diphenylpyrazolecarboxylic acid derivative of the formula I according to claim 1, together with inert carriers and adjuvants.

5. A method for the selective control of weeds in crops of useful plants, characterised in that the crops or the areas in which they are grown are treated with a composition according to claim 4.

6. A composition according to claim 3, characterised in that it comprises as herbicidally active component

a 2-[4-(7-chlorobenzoxazol-2-yloxy)-phenoxy]-propionic acid ester of the formula II

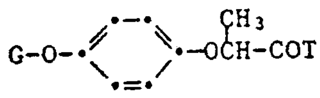

(II)

in which G represents a 7-chlorobenzoxazol-2-yl radical

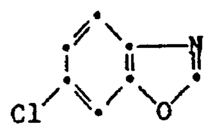

and T represents a radical selected from methoxy, ethoxy, propoxy, isopropoxy, butoxy, dimethylaminoethoxy, propargyloxy, 1-cyano-1-methyloxy, methoxycarbonylmethylthio, 1-ethoxycarbonylethoxy, butoxycarbonyl, acetoxim-oxy, methylethylketoxim-oxy and cyclohexanoximoxy, and, as safener, a 1,5-diphenylpyrazolecarboxylic acid derivative of the formula I, claim 1, together with inert carriers and adjuvants.

7. A method for the selective control of weeds in crops of useful plants, characterised in that the crops or the areas in which they are grown are treated with a composition according to claim 6.

8. A composition according to claim 3, characterised in that it comprises as herbicidally active component a 2-[4-(7-chloroquinoxalin-2-yloxy)-phenoxy]-propionic acid ester of the formula II

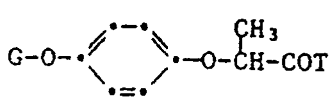

(II)

in which G represents the 7-chloroquinoxalin-2-yl radical

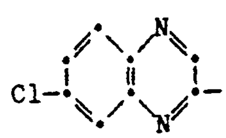

and T represents a radical selected from methoxy, ethoxy, propoxy, isopropoxy, butoxy, dimethylaminoethoxy, propargyloxy, 1-cyano-1-methylethoxy, methoxycarbonylmethylthio, 1-ethylcarbonylethoxy, butoxycarbonyl, acetoxim-oxy, methylethylketoxim-oxy and cyclohexanonoxim-oxy, and, as safener, a 1,5-diphenylpyrazolecarboxylic acid derivative of the formula I according to claim 1, together with inert carriers and adjuvants.

9. A method for the selective control of weeds in crops of useful plants, characterised in that the crops or the areas in which they are grown are treated with a composition according to claim 8.

10. A composition according to claim 3, characterised in that it comprises as herbicidally active component 2-[4-(7-chlorobenzoxazol-2-yloxy)-phenoxy]-propionic acid ethyl ester of the formula

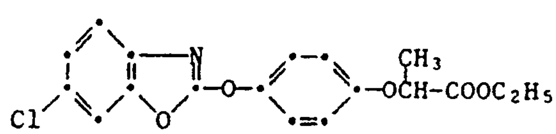

and, as safener, a 1,5-diphenylpyrazole-3-carboxylic acid derivative of the formula I according to claim 1, together with inert carriers and adjuvants.

11. A method for the selective control of weeds in crops of useful plants, characterised in that the crops or the areas in which they are grown are treated with a composition according to claim 10.

12. A composition according to claim 3, characterised in that it comprises as herbicidally active component 2-[4-(7-chloroquinoxalin-2-yloxy)-phenoxy]-propionic acid ethyl ester of the formula

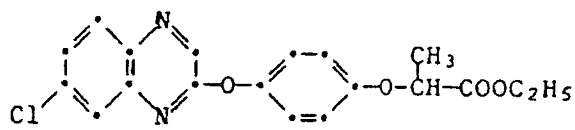

and, as safener, a 1,5-diphenylpyrazole-3-carboxylic acid derivative of the formula I according to claim 1, together with inert carriers and adjuvants.

13. A method for the selective control of weeds in crops of useful plants, characterised in that the crops or the areas in which they are grown are treated with a composition according to claim 12.

14. A composition according to claim 3, characterised in that it comprises as herbicidally active component 2-[4-(5-chloro-3-fluoropyridin-2-yloxy)-phenoxy]-propionic acid propargyl ester of the formula

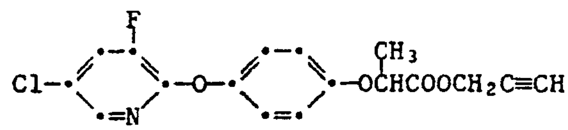

or its 2R enantiomer and, as safener, a 1,5-diphenylpyrazole-3-carboxylic acid derivative of the formula I according to claim 1, together with inert carriers and adjuvants.

15. A method for the selective control of weeds in crops of useful plants, characterised in that the crops or the areas in which they are grown are treated with a composition according to claim 14.

16. Novel 1,5-diphenylpyrazole-3-carboxylic acid derivatives of the formula (I)

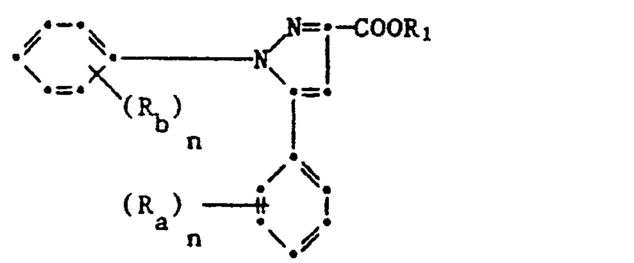

I

in which each of $R_a$ and $R_b$, independently of the other, represents halogen, $C_1$-$C_5$-alkyl, $C_1$-$C_5$-haloalkyl, $C_2$-$C_5$-alkenyl, $C_2$-$C_5$-alkynyl or cyano, n represents zero or a number from 1 to 3 and the group $-OR_1$ represents hydroxy, a plant-physiologically tolerable salt radical or any desired ester radical, in which

$R_1$ represents hydrogen, a plant-physiologically tolerable metal or ammonium cation, or $C_1$-$C_{18}$-alkyl or $C_3$-$C_{18}$-cycloalkyl each of which is unsubstituted or mono- or poly-substituted by $C_2$-$C_8$-alkenyl, $C_2$-$C_8$-alkynyl, $C_3$-$C_8$-cycloalkenyl, halogen, nitro, cyano or by an $-XR_{10}$, $-CXR_{10}$, $-CX-XR_{10}$, $-CXNR_3R_4$, $-XCXR_{10}$, $-XCXNR_3R_4$, $-NR_3R_4$, $-NR_3CONR_3R_4$, $-CONR_3-NR_3R_4$, $-CONR_3-CONR_3COR_4$, $-Si(C_1-C_4-alkyl)_3$, $-C(OR_7)(OR_8)OR_9$ or $PO(R_5)R_6$ radical; a heterocycle Q that is bonded via C or N; an unsubstituted or substituted phenyl or naphthyl radical U, a radical E-U or an imido radical $-N=C(R_2)$-

$R_2$,
$R_2$ represents $C_1$-$C_4$-alkyl or $C_3$-$C_7$-cycloalkyl, or the two radicals $R_2$ together represent a 4- to 6-membered $C_4$-$C_{12}$-alkylene radical that may be branched and/or interrupted by oxygen or sulphur,
each of $R_3$ and $R_4$, independently of the other, represents hydrogen, or $C_1$-$C_8$-alkyl or $C_3$-$C_8$-cycloalkyl each of which is unsubstituted or substituted by $C_2$-$C_6$-alkenyl,$C_2$-$C_6$-alkynyl, $C_3$-$C_7$-cycloalkyl, halogen, $C_1$-$C_8$-alkoxy, $C_2$-$C_8$-alkoxyalkoxy, $C_1$-$C_8$-alkylthio or by cyano, and one of $C_3$ and $C_4$ alternatively represents $C_1$-$C_8$-alkoxy, $-COOR_{10}$, $-CONH_2$, $CONH(C_1$-$C_4$-alkyl), $CON(R_2)R_2$, $-NH_2$, $-NH(C_1$-$C_2$-alkyl)-$N(R_2)R_2$, a heterocycle Q, or a phenyl or naphthyl radical U that is bonded to the nitrogen atom directly or via a $C_1$-$C_{14}$-alkylene bridge,
$R_3$ and $R_4$ together represent a 4- to 6-membered $C_4$-$C_{12}$-alkylene or -alkenylene chain that may be branched and/or interrupted by oxygen, sulphur, $N(C_1$-$C_4$-alkyl),N(benzyl), $-SO_2$-, -CO- or by $-C(OR_7)$-$OR_8$, and that may be substituted by halogen, $C_1$-$C_4$-alkyl, $C_1$-$C_4$-alkoxy, $-NH_2$, $-NH(C_1$-$C_4$-alkyl) or by $-N(C_1$-$C_4$-alkyl$)_2$,
each of $R_5$ and $R_6$, independently of the other, represents $C_1$-$C_4$-alkyl, hydroxy or $C_1$-$C_4$-alkoxy,
each of $R_7$ and $R_8$, independently of the other, represents $C_1$-$C_4$-alkyl,
$R_7$ and $R_8$ together represent $C_2$-$C_4$-alkylene,
each of $R_9$ and $R_{10}$ represents hydrogen, or $C_1$-$C_8$-alkyl or $C_3$-$C_8$-cycloalkyl each of which is unsubstituted or substituted by $C_2$-$C_6$-alkenyl, $C_2$-$C_6$-alkynyl, $C_3$-$C_6$-cycloalkenyl, halogen, $C_1$-$C_4$-alkoxy, $C_2$-$C_8$-alkoxyalkoxy, $C_1$-$C_6$-alkoxycarbonyl, $C_1$-$C_6$-haloalkoxycarbonyl, a heterocycle Q or by a phenyl or naphthyl radical U,
$R_9$ and $R_{10}$ also represent the phenyl or naphthyl radical U, or a heterocycle Q bonded via C,
Q represents a saturated or unsaturated 5- to 12-membered heterocycle that contains from 1 to 4 hetero atoms N, O, S or a -SO-, $-SO_2$-, $N(C_1$-$C_4$-allyl) or N(benzyl) group, wherein oxygen and sulphur must not be in vicinal positions, and that may be substituted by halogen, $C_1$-$C_4$-alkyl, $C_1$-$C_4$-alkoxy, $C_1$-$C_4$-haloalkyl, $C_1$-$C_4$-haloalkoxy, cyano or by nitro,
E represents a $C_1$-$C_4$-alkylene bridge, a $C_2$-$C_4$-alkenylene bridge or a $C_2$-$C_4$-alkynylene bridge,
U represents a phenyl or naphthyl radical that is unsubstituted or mono- or poly-substituted by halogen, $C_1$-$C_4$-alkyl, X $C_1$-$C_4$-alkyl, $C_1$-$C_4$-alkylsulphinyl, $C_1$-$C_4$-alkylsulphonyl, $C_1$-$C_4$-haloalkyl, $C_1$-$C_4$-haloalkoxy, cyano, nitro, -COOH, $-COOC_1$-$C_4$-alkyl, $-COC_1$-$C_4$-alkyl, $CONH_2$, $CONH(C_1$-$C_4$-alkyl), $CON(C_1$-$C_4$-alkyl$)_2$, $-SO_2NH_2$, $SO_2NH(C_1$-$C_4$-alkyl), $SO_2N(C_1$-$C_4$-alkyl), pyrrolidino, piperidino or by the pyrrolidinocarbonyl, piperidinocarbonyl or morpholinocarbonyl radical,
and X represents oxygen or sulphur,
with the proviso that, when $(R_a)$ is hydrogen, n = 1 and $R_1$ is hydrogen or ethyl, $(R_b)$, when it represents methyl or halogen, must be in the ortho position.

**17.** 1,5-diphenylpyrazole-3-carboxylic acid derivatives of the formula I, claim 16, characterised in that $R_a$, $R_b$ and n have the meanings given in claim 1, while $R_1$ represents hydrogen, a plant-physiologically tolerable metal or ammonium cation, or $C_1$-$C_{18}$-alkyl or $C_3$-$C_{18}$-cycloalkyl each of which is unsubstituted or mono- or poly-substituted by $C_2$-$C_8$-alkenyl, $C_2$-$C_8$-alkynyl, $C_3$-$C_8$-cycloalkenyl, halogen, nitro, cyano, $C_1$-$C_4$-alkoxy, $C_1$-$C_4$-alkoxycarbonyl, $C_1$-$C_4$-alkylcarbonyl, carbamoyl, di($C_1$-$C_4$-alkylcarbamoyl), amino, $C_1$-$C_4$-alkylamino or by di($C_1$-$C_4$-alkylamino), or $R_1$ represents a 5- or 6-membered heterocycle Q that is bonded via N or C, or a phenyl or naphthyl radical U, a radical E-U or an imido radical $-N=C(R_2)R_2$, E, $R_2$, Q and U having the meanings given in claim 16 with the proviso that, when $(R_a)$ is hydrogen, n = 1 and $R_1$ is hydrogen or ethyl, $(R_b)$, when it represents methyl or halogen, must be in the ortho position.

**18.** 1,5-diphenylpyrazole-3-carboxylic acid esters of the formula I, claim 16, in which $R_a$, $R_b$ and n have the meanings given in claim 1, while $R_1$ represents $C_1$-$C_{18}$-alkyl that is unsubstituted or mono- or poly-substituted by $C_2$-$C_8$-alkyl, $C_2$-$C_8$-alkynyl, halogen, nitro, cyano, $C_1$-$C_4$-alkoxy or by phenyl.

**19.** 1,5-diphenylpyrazole-3-carboxylic acid salts of the formula I, claim 16, in which $R_a$, $R_b$ and n have the meanings given in claim 1 and $R_1$ represents a plant-physiologically tolerable metal or ammonium cation.

**20.** 1-(2-chlorophenyl)-3-methoxycarbonyl-5-phenylpyrazole according to claim 16.

**21.** 1-(2,4-dichlorophenyl)-3-methoxycarbonyl-5-phenylpyrazole according to claim 16.

22. 1-(2-chlorophenyl)-3-methoxycarbonyl-5-(2-fluorophenyl)-pyrazole according to claim 16.

23. A process for the preparation of the 1,5-diphenylpyrazole-3-carboxylic acids and derivatives of the formula I according to claim 16, characterised in that an acetophenone of the formula III

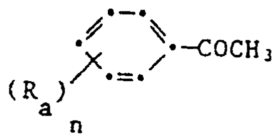 (III)

in which $R_a$ and n have the meanings given under formula I is reacted in an inert organic solvent in the presence of a base with an equimolar amount of an oxalic acid diester of the formula IV

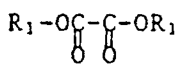 (IV)

in which $R_1$ has the meaning given under formula I, there is added to the resulting benzoylpyruvic acid ester of the formula V

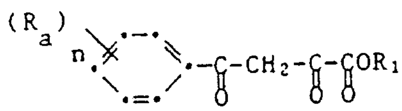 (V)

in which $R_a$, $R_1$ and n have the meanings given under formula I an equimolar amount of phenylhydrazine of the formula VI

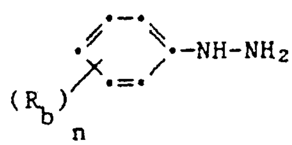 (VI)

in which $R_b$ and n have the meanings given under formula I, and then the mixture is cyclised in an acidic medium at elevated temperature to form the 1,5-diphenylpyrazolecarboxylic acid derivative of the formula I.

24. A process for the preparation of the 1,5-diphenylpyrazole-3-carboxylic acid derivatives of the formula I according to claim 16, characterised in that a 1,5-diphenylpyrazole-3-carboxylic acid halide of the formula VII

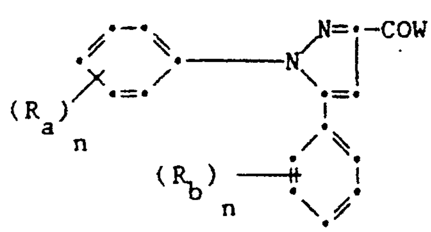 (VII),

in which n, $R_a$ and $R_b$ have the meanings given in claim 16 and W represents a halogen atom chlorine or bromine, is reacted in an inert solvent or diluent with an alcohol of the formula VIII or a metal or ammonium salt thereof

$R_1OH$ (VIII)

in which $R_1$ has the meaning given in claim 16.

**25.** Novel 1,5-diphenylpyrazole-3-carboxylic acid halides of the formula VII

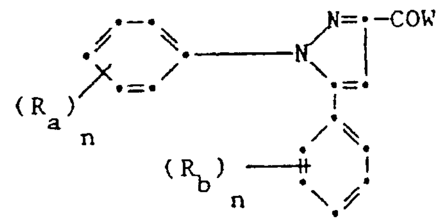

(VII),

in which n, $R_a$ and $R_b$ have the meanings given in claim 16 and W represents a halogen atom chlorine or bromine, as intermediates for the preparation of the 1,5-diphenylpyrazole-3-carboxylic acid derivatives according to claim 16.

## Revendications

**1.** Utilisation des dérivés de l'acide 1,5-diphénylpyrazol-3-carboxylique pour la protection des plantes cultivées contre l'action phytotoxique des esters des acides 2-[4-(phényl-, pyridin-2-yl-, benzoxazol-, benzothiazol- et quinoxalin-2-yl-oxy)-phénoxy]propioniques actifs comme herbicides, caractérisé en ce que l'on traite les plantes cultivées, des parties de ces plantes ou les surfaces cultivées avec un dérivé de l'acide 1,5-diphénylpyrazol-3-carboxylique de formule I

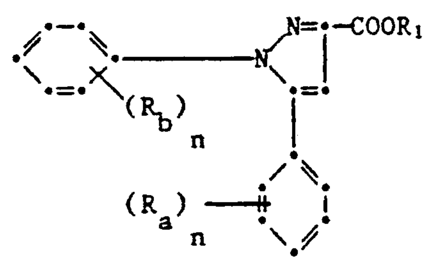

I

où $R_a$ et $R_b$ représentent, indépendamment l'un de l'autre, un halogène, un alkyle en $C_1$-$C_5$, un halogénoalkyle en $C_1$-$C_5$, un alcényle en $C_2$-$C_5$, un alcynyle en $C_2$-$C_5$ ou un cyano, n est égal à 0 ou est un nombre allant de 1 à 3 et où le groupe $-OR_1$ représente l'hydroxy, un reste de sel ou un reste d'ester quelconque physiologiquement compatible avec les plantes,
$R_1$ représentant l'hydrogène, un cation métallique ou un cation ammonium physiologiquement compatible avec les plantes, un reste alkyle en $C_1$-$C_{18}$ ou cycloalkyle en $C_3$-$C_{18}$ non substitué ou substitué une ou plusieurs fois par un alcényle en $C_2$-$C_8$, un alcynyle en $C_2$-$C_8$, un cycloalcényle en $C_3$-$C_8$, un halogène, un nitro, un cyano, un reste $-XR_{10}$, $-CXR_{10}$, $-CX-XR_{10}$, $-CXNR_3R_4$, $-XCXR_{10}$, $-XCXNR_3R_4$, $-NR_3R_4$- $-NR_3CONR_3R_4$, $-CONR_3-NR_3R_4$, $-CONR_3-CONR_3R_4$, Si(alkyle en $C_1$-$C_4$)$_3$, $-C(OR_7)(OR_8)OR_9$ $PO(R_5)R_6$, un hétérocycle Q lié par C ou N, un reste U phényle ou naphtyle non substitué ou substitué, un reste E-U ou un reste imido $-N=C(R_2)R_2$,
$R_2$ représente un alkyle en $C_1$-$C_4$, un cycloalkyle en $C_3$-$C_7$ ou les deux $R_2$ représentent ensemble un reste alkylène en $C_4$-$C_{12}$ à 4-6 chaînons, pouvant être ramifié et/ou interrompu par l'oxygène ou le soufre,
$R_3$ et $R_4$ représentent, indépendamment l'un de l'autre, l'hydrogène, un alkyle en $C_1$-$C_8$ ou un cycloalkyle en $C_3$-$C_8$, non substitué ou substitué par un alcényle en $C_2$-$C_6$, un alcynyle en $C_2$-$C_6$, un cycloalkyle en $C_3$-$C_7$, un halogène, un alcoxy en $C_1$-$C_8$, un alcoxyalcoxy en $C_2$-$C_8$, un alkyle en $C_1$-$C_8$ thio, un cyano, un alcoxy en $C_3$ et $C_4$ également un alcoxy en $C_1$-$C_8$, $-COOR_{10}$, $-CONH_2$, CONH(alkyle en $C_1$-$C_4$), $CON(R_2)R_2$, $-NH_2$, -NH(alkyle en $C_1$-$C_2$)-$N(R_2$-$R_2$, un hétérocycle Q, un reste U phényle ou naphtyle directement lié à l'atome d'azote ou lié à l'atome d'azote par un pont alkylène en $C_1$-$C_{14}$,
$R_3$ et $R_4$ représentent ensemble une chaîne alkylène ou alcénylène en $C_4$-$C_{12}$ à 4-6 chaînons, pouvant

être ramifiée et/ou interrompue par l'oxygène ou le soufre, N(alkyle en $C_1$-$C_4$), N(benzyle), -$SO_2$- ou -CO- ou $C(OR_7)$ $OR_8$ et pouvant être substituée par un halogène, un alkyle en $C_1$-$C_4$, un alcoxy en $C_1$-$C_4$, -$NH_2$, -NH(alkyle en $C_1$-$C_4$), -N(alkyle en $C_1$-$C_4$)$_2$,

$R_5$ et $R_6$ représentent, indépendamment l'un de l'autre, un alkyle en $C_1$-$C_4$, un hydroxy ou un alcoxy en $C_1$-$C_4$,

$R_7$ et $R_8$ représentent, indépendamment l'un de l'autre, un alkyle en $C_1$-$C_4$,

$R_7$ et $R_8$ représentent ensemble un alkylène en $C_2$-$C_4$,

$R_9$ et $R_{10}$ représentent l'hydrogène, un alkyle en $C_1$-$C_8$ ou un cycloalkyle en $C_3$-$C_8$, non substitué ou substitué par un alcényle en $C_2$-$C_6$, un alcynyle en $C_2$-$C_6$ ou un cycloalcényle en $C_3$-$C_6$, un halogène, un alcoxy en $C_1$-$C_4$, un alcoxyalcoxy en $C_2$-$C_8$, un alcoxy en $C_1$-$C_6$ carbonyle, un haloalcoxy en $C_1$-$C_6$ carbonyle, un hétérocycle Q ou un reste U phényle ou naphtyle,

$R_9$ et $R_{10}$ représentent, en outre, le reste U phényle ou naphtyle ou un hétérocycle Q lié par C,

Q représente un hétérocycle à 5-12 chaînons saturé ou insaturé contenant 1 à 4 hétéroatomes N, O, S respectivement un groupe -SO-, -$SO_2$-, N(alkyle en $C_1$-$C_4$) ou un groupe -N(benzyle), l'oxygène et le soufre ne devant pas se trouver en position vicinale et pouvant être substitué par un halogène, un alkyle en $C_1$-$C_4$, un alcoxy en $C_1$-$C_4$, un haloalkyle en $C_1$-$C_4$, un haloalcoxy en $C_1$-$C_4$, un cyano, un nitro,

E représente un pont alkylène en $C_1$-$C_4$, un pont alcénylène en $C_2$-$C_4$ ou un pont alcynylène en $C_2$-$C_4$,

U représente un reste phényle ou naphtyle, non substitué ou substitué une ou plusieurs fois par un halogène, un alkyle en $C_1$-$C_4$, X alkyle en $C_1$-$C_4$, un alkyle en $C_1$-$C_4$ sulfinyle, un alkyle en $C_1$-$C_4$ sulfonyle, un haloalkyle en $C_1$-$C_4$, un haloalcoxy en $C_1$-$C_4$, un cyano, un nitro, -COOH, -COO alkyle en $C_1$-$C_4$, -CO alkyle en $C_1$-$C_4$, $CONH_2$, CONH(alkyle en $C_1$-$C_4$), CON (alkyle en $C_1$-$C_4$)$_2$, -$SO_2NH_2$, $SO_2$NH(alkyle en $C_1$-$C_4$), $SO_2$N(alkyle en $C_1$-$C_4$), le pyrrolidino, le pipéridino ou le pyrrolidinocarbonyle, le pipéridinocarbonyle, le reste morpholinocarbonyle,

X représente l'oxygène ou le soufre,

et en ce que l'on traite la culture ou la surface cultivée simultanément ou successivement avec un ester des acides 2-[4-(phényloxy, pyridin-2-yloxy, benzoxazolyloxy, benzothiazolyloxy ou quinoxalin-2-yloxy)-phénoxy]propioniques de formule II

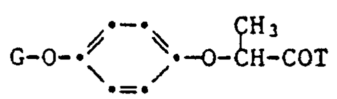 (II),

où G représente

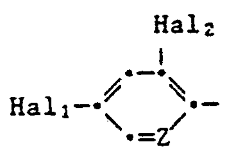
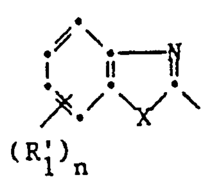 ou 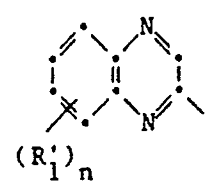 ,

$Hal_1$ représente le fluor, le chlore, le brome, l'iode ou le trifluorométhyle, $Hal_2$ représente l'hydrogène, le fluor, le chlore, le brome ou le trifluorométhyle, Z représente l'azote ou le méthine (-CH=), X représente un atome d'oxygène ou de soufre, $R_1'$ représente un halogène, le trifluorométhyle, un nitro, un cyano, un alkyle en $C_1$-$C_4$ ou un alcoxy en $C_1$-$C_4$ et n représente 0, 1, 2 ou 3 et

T représente

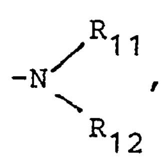 , 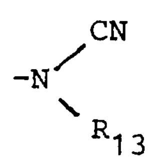 ,

-$OR_{14}$ ou -O-N=$CR_{15}R_{16}$,

$R_{11}$, $R_{12}$ représentant, indépendamment l'un de l'autre, l'hydrogène, un alcoxy en $C_1$-$C_8$, un alkyle en

$C_1$-$C_8$, le phényle ou le benzyle, $R_{11}$ et $R_{12}$ représentent ensemble avec l'atome d'azote les portant, un hétérocycle azoté saturé ayant 5 à 6 chaînons, pouvant être interrompu par un atome d'oxygène ou de soufre,

$R_{13}$ représentant un alkyle en $C_1$-$C_4$, un alcényle en $C_3$-$C_4$, un alcynyle en $C_3$-$C_4$ ou un alcoxy en $C_2$-$C_4$ alkyle,

$R_{14}$ représentant l'hydrogène ou l'équivalent d'un ion alcalin, alcalino-terreux, cuivre ou fer; un reste alkyle en $C_1$-$C_4$ ammonium quaternaire ou hydroxyalkyle en $C_1$-$C_4$ ammonium quaternaire; un reste alkyle en $C_1$-$C_9$ éventuellement substitué une ou plusieurs fois par un amino, un halogène, un hydroxyle, un cyano, un nitro, un phényle, un alcoxy en $C_2$-$C_4$, un polyéthoxy ayant 2 à 6 unités d'oxyde d'éthylène, -COOR, -COSR, -$CONH_2$-, -CON(alcoxy en $C_1$-$C_4$)alkyle en $C_1$-$C_4$, -CO-N-dialkyle en $C_1$-$C_4$, -CONH alkyle en $C_1$-$C_4$, -N(alcoxy en $C_1$-$C_4$)alkyle en $C_1$-$C_4$ ou dialkyle en $C_1$-$C_4$ amino; un reste alcényle en $C_3$-$C_9$ éventuellement substitué par un halogène ou par un alcoxy en $C_1$-$C_4$; un reste alcynyle en $C_3$-$C_9$ éventuellement substitué par un halogène ou un alcoxy en $C_1$-$C_4$; un cycloalkyle en $C_3$-$C_9$; ou un phényle éventuellement substitué par un cyano, un alkyle en $C_1$-$C_4$, un alcoxy en $C_1$-$C_4$, un acétyle, -$COOR_{17}$, -$COSR_{17}$, -$CONH_2$, -CON(alcoxy en $C_1$-$C_4$) alkyle en $C_1$-$C_4$, un -CO-N-dialkyle en $C_1$-$C_4$ ou un -CONH alkyle en $C_1$-$C_4$ et

$R_{16}$ représentant, indépendamment l'un de l'autre, un alkyle en $C_1$-$C_4$ ou représente ensemble une chaîne alkylène ayant 3 à 6 chaînons et

$R_{17}$ représente l'hydrogène, un alkyle en $C_1$-$C_6$, un halogénoalkyle en $C_1$-$C_6$, un alcoxy en $C_2$-$C_6$ alkyle, un alcényle en $C_3$-$C_6$, un halogénoalcényle en $C_3$-$C_6$, un alcynyle en $C_3$-$C_6$ ou un halogénoalcynyle en $C_3$-$C_6$.

**2.** Procédé pour lutter contre les mauvaises herbes dans les cultures de plantes utiles, caractérisé en ce que l'on traite les cultures ou les surfaces cultivées simultanément ou successivement après un court intervalle de temps avec une quantité efficace en tant qu'herbicide d'un ester des acides 2-[4-phényloxy, pyridin-2-yloxy, benzoxazolyloxy, benzothiazolyloxy ou quinoxalin-2-yl-oxy)phénoxy]-propioniques de formule II selon la revendication 1 et avec, comme antidote, une quantité efficace d'un dérivé de l'acide 1,5-diphénylpyrazol-3-carboxylique de formule I selon la revendication 1.

**3.** Agent herbicide sélectif pour lutter contre les mauvaises herbes dans les cultures de plantes utiles, caractérisé en ce qu'il contient, comme composant actif en tant qu'herbicide, un ester des acides 2-[4-(phényloxy, pyridin-2-yloxy, benzoxazolyloxy, benzothiazolyloxy ou quinoxalin-2-yloxy)phénoxy]-propioniques de formule II selon la revendication 1, et comme antidote, une quantité efficace d'un dérivé de l'acide 1,5-diphénylpyrazol-3-carboxylique de formule I selon la revendication 1, en association avec un support inerte et des adjuvants.

**4.** Agent selon la revendication 3, caractérisé en ce qu'il contient, comme composant actif en tant qu'herbicide, un ester de l'acide 4-(4-chloro-2-fluoropyridin-2-yloxy)phénoxy-propionique de formule II.

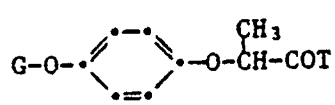

(II),

où G représente le reste 4-chloro-2-fluoropyridin-2-yle

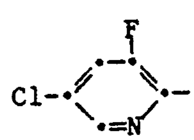

et T représente un reste choisi parmi le méthoxy, l'éthoxy, le propyloxy, l'isopropyloxy, le butyloxy, le diméthylaminoéthyloxy, le propargyloxy, le 1-cyano-1-méthyléthoxy, le méthylcarbonylméthylthio, le 1-éthoxycarbonyléthoxy, le butoxycarbonyle, l'acétoximoxy, le méthyléthylcétoximoxy, le cyclohexanoximoxy et, comme antidote, un dérivé de l'acide 1,5-diphénylpyrazol-3-carboxylique de formule I selon la revendication 1, en association avec un support inerte et des adjuvants.

5. Procédé pour lutter sélectivement contre les mauvaises herbes dans les cultures de plantes utiles, caractérisé en ce que l'on traite les cultures ou les surfaces cultivées avec un agent selon la revendication 4.

6. Agent selon la revendication 3, caractérisé en ce qu'il contient, comme composant actif en tant qu'herbicide, un ester de l'acide 2-[4-(7-chlorobenzoxazol-2-yloxy)phénoxy]propionique de formule II

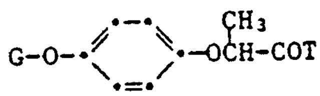

(II)

où G représente le reste 7-chlorobenzoxazol-2-yle

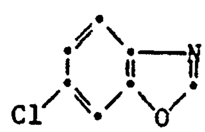

et T représente un reste choisi parmi le méthoxy, l'éthoxy, le propyloxy, l'isopropyloxy, le butyloxy, le diméthylaminoéthyloxy, le propargyloxy, le 1-cyano-1-méthyloxy, le méthoxycarbonylméthylthio, le 1-éthoxycarbonyléthoxy, le butoxycarbonyle, l'acétoximoxy, le méthyléthylcétoximoxy et le cyclohexanoximoxy et, comme antidote, un dérivé de l'acide 1,5-diphénylpyrazolcarboxylique de formule I selon la revendication 1, en association avec un support inerte et des adjuvants.

7. Procédé pour lutter sélectivement contre les mauvaises herbes dans les cultures de plantes utiles, caractérisé en ce que l'on traite les cultures ou les surfaces cultivées avec un agent selon la revendication 6.

8. Agent selon la revendication 3, caractérisé en ce qu'il contient, comme composant actif en tant qu'herbicide, un ester de l'acide 2-[4-(7-chloroquinoxalin-2-yloxy)-phénoxy]-propionique de formule II

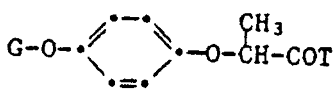

(II)

où G représente le reste 7-chloroquinoxalin-2-yle

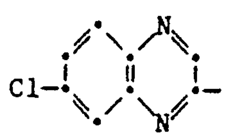

et T représente un reste choisi parmi le méthoxy, l'éthoxy, le propyloxy, l'isopropyloxy, le butyloxy, le diméthylaminoéthoxy, le propargyloxy, le 1-cyano-1-méthyléthoxy, le méthoxycarbonylméthylthio, le 1-éthylcarbonyléthoxy, le butyloxycarbonyle, l'acétoximoxy, le méthyléthylcétoximoxy, et le cyclohexanonoximoxy et, comme antidote, un dérivé de l'acide 1,5-diphénylpyrazolcarboxylique de formule I selon la revendication 1, en association avec un support inerte et des adjuvants.

9. Procédé pour lutter sélectivement contre les mauvaises herbes dans les cultures de plantes utiles, caractérisé en ce que l'on traite les cultures ou les surfaces cultivées avec un agent selon la revendication 8.

10. Agent selon la revendication 3, caractérisé en ce qu'il contient, comme composant actif en tant qu'herbicide, l'ester éthylique de l'acide 2-[4-(7-chlorobenzoxazol-2-yloxy)-phénoxy]-propionique de

formule

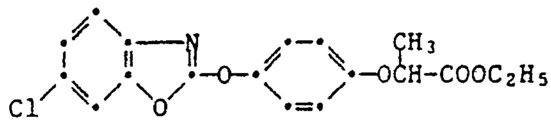

et, comme antidote, un dérivé de l'acide 1,5-diphénylpyrazol-3-carboxylique de formule I selon la revendication 1, en association avec un support inerte et des adjuvants.

**11.** Procédé pour lutter sélectivement contre les mauvaises herbes dans les cultures de plantes utiles, caractérisé en ce que l'on traite les cultures ou les surfaces cultivées avec un agent selon la revendication 10.

**12.** Agent selon la revendication 3, caractérisé en ce qu'il contient, comme composant actif en tant qu'herbicide, l'ester éthylique de l'acide 2-[4-(7-chloroquinoxalin-2-yloxy)-phénoxy]-propionique de formule

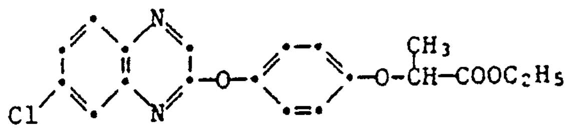

et, comme antidote, un dérivé de l'acide 1,5-diphénylpyrazol-3-carboxylique de formule I selon la revendication 1, en association avec un support inerte et des adjuvants.

**13.** Procédé pour lutter sélectivement contre les mauvaises herbes dans les cultures de plantes utiles, caractérisé en ce que l'on traite les cultures ou les surfaces cultivées avec un agent selon la revendication 12.

**14.** Agent selon la revendication 3, caractérisé en ce qu'il contient, comme composant actif en tant qu'herbicide, l'ester propargylique de l'acide 2-[4-(5-chloro-3-fluoropyridin-2-yloxy)-phénoxy]-propionique de formule

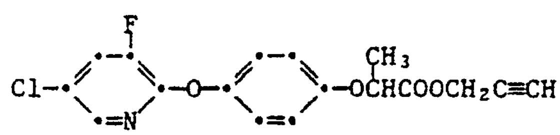

ou de son énantiomère 2 R et, comme antidote, un dérivé de l'acide 1,5-diphénylpyrazol-3-carboxylique de formule I selon la revendication 1, en association avec un support inerte et des adjuvants.

**15.** Procédé pour lutter sélectivement contre les mauvaises herbes dans les cultures de plantes utiles, caractérisé en ce que l'on traite les cultures ou les surfaces cultivées avec un agent selon la revendication 14.

**16.** Nouveaux dérivés de l'acide 1,5-diphénylpyrazol-3-carboxylique de formule (1)

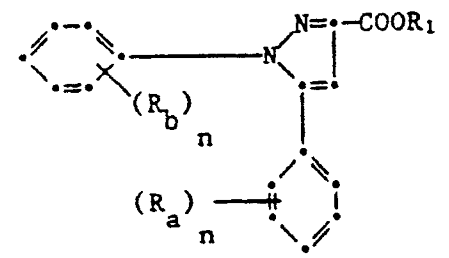

I

où $R_a$ et $R_b$ représentent, indépendamment l'un de l'autre, un halogène, un alkyle en $C_1$-$C_5$, un halogénoalkyle en $C_1$-$C_5$, un alcényle en $C_2$-$C_5$, un alcynyle en $C_2$-$C_5$ ou un cyano, n est égal à 0 ou est un nombre allant de 1 à 3 et où le groupe -$OR_1$ représente l'hydroxy ou un reste de sel ou un reste d'ester quelconque physiologiquement compatible avec les plantes.

$R_1$ représentant l'hydrogène, un cation métallique ou un cation ammonium physiologiquement compatible avec les plantes, un reste alkyle en $C_1$-$C_{18}$ ou cycloalkyle en $C_3$-$C_{18}$ non substitué ou substitué une ou plusieurs fois par un alcényle en $C_2$-$C_8$, un alcynyle en $C_2$-$C_8$, un cycloalcényle en $C_3$-$C_8$, un halogène, un nitro, un cyano, un reste -$XR_{10}$, -$CXR_{10}$, -CX-$XR_{10}$, -$CXNR_3R_4$, -$XCXR_{10}$, -$XCXNR_3R_4$, -$NR_3R_4$, -$NR_3CONR_3R_4$, -$CONR_3$-$NR_3R_4$, -$CONR_3$-$CONR_3COR_4$, Si(alkyle en $C_1$-$C_4$)$_3$, -C($OR_7$)($OR_8$)-$OR_9$ PO($R_5$)$R_6$, un hétérocycle Q lié par C ou N; un reste U phényle ou naphtyle non substitué ou substitué, un reste E-U ou un reste imido -N = C($R_2$)$R_3$,

$R_2$ représente un alkyle en $C_1$-$C_4$, un cycloalkyle en $C_3$-$C_7$ ou les deux $R_2$ représentent ensemble un reste alkylène en $C_4$-$C_{12}$ à 4-6 chaînons, pouvant être ramifié et/ou interrompu par l'oxygène ou le soufre,

$R_3$ et $R_4$ représentent, indépendamment l'un de l'autre, l'hydrogène, un alkyle en $C_1$-$C_8$ ou un cycloalkyle en $C_3$-$C_8$, non substitué ou substitué par un alcényle en $C_2$-$C_6$, un alcynyle en $C_2$-$C_6$, un cycloalkyle en $C_3$-$C_7$, un halogène, un alcoxy en $C_1$-$C_8$, un alcoxyalcoxy en $C_2$-$C_8$, un alkyle en $C_1$-$C_8$ thio, un cyano, un alcoxy en $C_3$ et $C_4$ également un alcoxy en $C_1$-$C_8$, -$COOR_{10}$, -$CONH_2$, CONH(alkyle en $C_1$-$C_4$), CON($R_2$)$R_2$, -$NH_2$, -NH(alkyle en $C_1$-$C_2$)-N($R_2$)$R_2$, un hétérocycle Q, un reste U phényle ou naphtyle directement lié à l'atome d'azote ou lié à l'atome d'azote par un pont alkylène en $C_1$-$C_{14}$,

$R_3$ et $R_4$ représentent ensemble une chaîne alkylène ou alcénylène en $C_4$-$C_{12}$ à 4-6 chaînons, pouvant être ramifiée et/ou interrompue par l'oxygène ou le soufre, N(alkyle en $C_1$-$C_4$), N(benzyle), -$SO_2$- ou -CO- ou -C($OR_7$)$OR_8$ et pouvant être substituée par un halogène, un alkyle en $C_1$-$C_4$, un alcoxy en $C_1$-$C_4$, -$NH_2$, -NH(alkyle en $C_1$-$C_4$), -N(alkyle en $C_1$-$C_4$)$_2$,

$R_5$ et $R_6$ représentent, indépendamment l'un de l'autre, un alkyle en $C_1$-$C_4$, un hydroxy ou un alcoxy en $C_1$-$C_4$,

$R_7$ et $R_8$ représentent, indépendamment l'un de l'autre, un alkyle en $C_1$-$C_4$,

$R_7$ et $R_8$ représentent ensemble un alkylène en $C_2$-$C_4$,

$R_9$ et $R_{10}$ représentent l'hydrogène, un alkyle en $C_1$-$C_8$ ou un cycloalkyle en $C_3$-$C_8$, non substitué ou substitué par un alcényle en $C_2$-$C_6$, un alcynyle en $C_2$-$C_6$ ou un cycloalcényle en $C_3$-$C_6$, un halogène, un alcoxy en $C_1$-$C_4$, un alcoxyalcoxy en $C_2$-$C_8$, un alcoxy en $C_1$-$C_6$ carbonyle, un haloalcoxy en $C_1$-$C_6$ carbonyle, un hétérocycle Q ou un reste U phényle ou naphtyle,

$R_9$ et $R_{10}$ représentent, en outre, le reste U phényle ou naphtyle ou un hétérocycle Q lié par C,

Q représente un hétérocycle à 5-12 chaînons saturé ou insaturé contenant 1 à 4 hétéroatomes N, O, S respectivement un groupe -SO-, -$SO_2$- -N(alkyle en $C_1$-$C_4$) ou un groupe -N(benzyle), l'oxygène et le soufre ne devant pas se trouver en position vicinale et pouvant être substitué par un halogène, un alkyle en $C_1$-$C_4$, un alcoxy en $C_1$-$C_4$, un haloalkyle en $C_1$-$C_4$, un haloalcoxy en $C_1$-$C_4$, un cyano, un nitro,

E représente un pont alkylène en $C_1$-$C_4$, un pont alcénylène en $C_2$-$C_4$ ou un pont alcynylène en $C_2$-$C_4$,

U représente un reste phényle ou naphtyle, non substitué ou substitué une ou plusieurs fois par un halogène, un alkyle en $C_1$-$C_4$, X alkyle en $C_1$-$C_4$, un alkyle en $C_1$-$C_4$ sulfinyle, un alkyle en $C_1$-$C_4$ sulfonyle, un haloalkyle en $C_1$-$C_4$, un haloalcoxy en $C_1$-$C_4$, un cyano, un nitro, -COOH, -COO alkyle en $C_1$-$C_4$, -CO alkyle en $C_1$-$C_4$, $CONH_2$, CONH(alkyle en $C_1$-$C_4$), CON (alkyle en $C_1$-$C_4$)$_2$, -$SO_2NH_2$,$SO_2$NH(alkyle en $C_1$-$C_4$), $SO_2$N (alkyle en $C_1$-$C_4$), le pyrrolidino, le pipéridino ou le pyrrolidinocarbonyle, le pipéridinocarbonyle, le reste morpholinocarbonyle,

X représente l'oxygène ou le soufre,

sous réserve que, lorsque ($R_a$) représente l'hydrogène, n = 1 et $R_1$ est l'hydrogène ou l'éthyle, et que, lorsque ($R_b$) représente le méthyle ou un halogène, ($R_b$) est dans la position ortho.

**17.** Dérivés de l'acide 1,5-diphénylpyrazol-3-carboxylique de formule I selon la revendication 16, caractérisés en ce que $R_a$, $R_b$ et n ont la signification donnée dans la revendication 1, tandis que $R_1$ représente l'hydrogène, un cation métallique ou ammonium physiologiquement compatible avec les plantes, un alkyle en $C_1$-$C_{18}$ ou un cycloalkyle en $C_3$-$C_{18}$ non substitué ou une ou plusieurs fois substitué par un alcényle en $C_2$-$C_8$, un alcynyle en $C_2$-$C_8$, un cycloalcényle en $C_3$-$C_8$, un halogène, un nitro, un cyano, un alcoxy en $C_1$-$C_4$, un alcoxy en $C_1$-$C_4$ carbonyle, un alkyle en $C_1$-$C_4$ carbonyle, un carbamoyle, un di(alkyle en $C_1$-$C_4$ carbamoyle), un amino, un alkyle en $C_1$-$C_4$ amino, un di(alkyle en $C_1$-$C_4$ amino), un hétérocycle Q à 5-6 chaînons lié par N ou C ou un reste U phényle ou naphtyle, un reste E-U ou un reste imido $-N=C(R_2)R_2$, E, $R_2$, Q et U ayant la signification donnée dans la revendication 16, sous réserve que lorsque ($R_a$) représente l'hydrogène, n=1 et $R_1$ est l'hydrogène ou l'éthyle, que lorsque ($R_b$) représente le méthyle ou un halogène, ($R_b$) est en position ortho.

**18.** Esters de l'acide 1,5-diphénylpyrazol-3-carboxylique de formule I selon la revendication 16 où $R_a$, $R_b$ et n ont la signification donnée dans la revendication 1, tandis que $R_1$ représente un alkyle en $C_1$-$C_{18}$ non substitué ou une ou plusieurs fois substitué par un alkyle en $C_2$-$C_8$, un alcynyle en $C_2$-$C_8$, un halogène, un nitro, un cyano, un alcoxy en $C_1$-$C_4$ ou un phényle.

**19.** Sels de l'acide 1,5-diphénylpyrazol-3-carboxylique de formule I selon la revendication 16 où $R_a$, $R_b$ et n ont la signification donnée dans la revendication 1 et $R_1$ représente un cation métallique ou ammonium physiologiquement compatible avec les plantes.

**20.** Le 1-(2-chlorophényl)-3-méthoxycarbonyl-5-phénylpyrazole selon la revendication 16.

**21.** Le 1-(2,4-dichlorophényl)-3-méthoxycarbonyl-5-phénylpyrazole selon la revendication 16.

**22.** Le 1-(2-chlorophényl)-3-méthoxycarbonyl-5-(2-fluorophényl)-pyrazole selon la revendication 16.

**23.** Procédé pour la préparation des acides 1,5-diphénylpyrazol-3-carboxyliques et dérivés de formule I selon la revendication 16, caractérisé en ce que l'on fait réagir une acétophénone de formule III

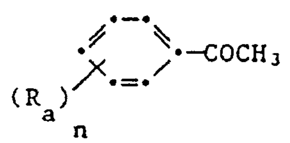

(III)

où $R_a$ et n ont la signification donnée pour la formule I, dans un solvant organique inerte en présence d'une base avec la quantité équimolaire d'un diester de l'acide oxalique de formule IV

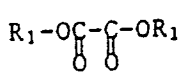

(IV)

où $R_1$ a la signification donnée pour la formule I, et en ce que l'on additionne l'ester de l'acide benzoylpyruvique de formule V formé

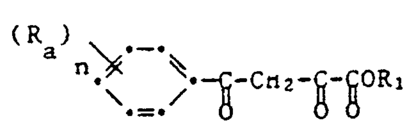

(V)

où $R_a$, $R_1$ et n ont la signification donnée pour la formule I d'une quantité équimolaire de phénylhydrazine de formule VI

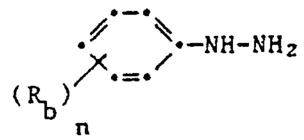

(VI)

où $R_b$ et n ont la signification donnée pour la formule I, le mélange étant ensuite cyclisé en milieu acide à une température augmentée en dérivé de l'acide 1,5-diphénylpyrazolcarboxylique de formule I.

**24.** Procédé pour la préparation des dérivés de l'acide 1,5-diphénylpyrazol-3-carboxylique de formule I selon la revendication 16, caractérisé en ce que l'on fait réagir, dans un solvant ou diluant inerte, un halogénure de l'acide 1,5-diphénylpyrazol-3-carboxylique de formule VII

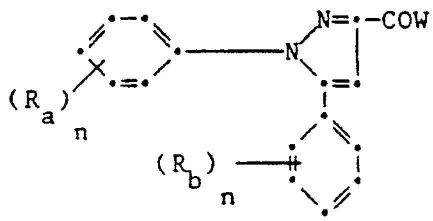

(VII),

où n, $R_a$ et $R_b$ ont la signification donnée dans la revendication 16 et W représente un atome d'halogène, le chlore ou le brome, avec un alcool de formule VIII ou un sel métallique ou d'ammonium de celui-ci

$R_1OH$ (VIII)

où $R_1$ a la signification donnée dans la revendication 16.

**25.** Nouveaux halogénures de l'acide 1,5-diphé-nylpyrazol-3-carboxylique de formule VII

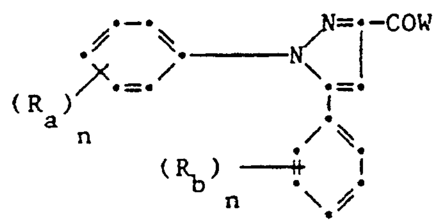

(VII),

où n, $R_a$ et $R_b$ ont la signification donnée dans la revendication 16 et W représente un atome d'halogène, le chlore ou le brome, comme produits intermédiaires pour la préparation des dérivés de l'acide 1,5-diphénylpyrazol-3-carboxylique selon la revendication 16.